# EUROPEAN PATENT SPECIFICATION

(11) **EP 4 556 422 B1**
(45) Date of publication and mention of the grant of the patent: **27.05.2026**
(21) Application number: 24211200.1
(22) Date of filing: 06.11.2024
(51) Int. Cl.: B65H 63/06, G01N 21/89, G01N 33/36

(54) **HOUSING OF A YARN CLEARER AND CORRESPONDING METHOD OF ASSEMBLY**
GEHÄUSE EINES GARNREINIGERS UND ZUGEHÖRIGES MONTAGEVERFAHREN
BOÎTIER D'UN ÉPURATEUR DE FIL ET PROCÉDÉ DE MONTAGE CORRESPONDANT

(30) Priority: 16.11.2023 LU 505548
(43) Date of publication of application: 21.05.2025
(73) Proprietor: Saurer Intelligent Technology AG, 9320 Arbon (CH)
(72) Inventor: Dumoulin, Charles Leopold Elisabeth, 9436 Balgach (CH); Adili, Ilir, 9443 Widnau (CH)
(74) Representative: Morgenthum-Neurode, Mirko

(56) References cited:
- EP-A1- 4 234 466
- EP-A2- 1 655 599
- EP-B1- 1 302 426
- DE-A1- 102008 000 610
- DE-B- 1 276 529
- FR-A- 1 443 425

## Description

The invention refers to a housing. Furthermore, the invention refers to a clearer. The invention also refers to a textile machine. The invention refers to a method for assembling a housing.

Clearer are known in the art e.g., from EP 1 655 599 B1. Therein, a clearer comprises a yarn sensor for optically scanning a yarn moving longitudinally in a measuring gap supplied with light rays from a source with a first receiver for directly transmitted light, two further receivers for light reflected from the yarn and elements for transmitting light between the source, gap and receivers. The transmitting element between the source and gap includes a diffusing foil, an aperture, and a lens behind the source in the radiation direction, such that the aperture is imaged at infinity. The transmitting elements between the gap and reflected light receivers each contain a lens before the receiver in the radiation direction, such that an image discernable by each receiver lies, in the absence of yarn on the opposite wall of the measurement gap, such that each image of the reflected light receivers lies crosswise on the other side and outside of the image of the source on the wall.

The clearer in the art provides a complex and difficult to handle optical setup in combination with a complex housing which limits the potential of the clearer with respect to the optical setup capabilities. Furthermore, the clearer in the art is complex in assemblance and therefore tedious to assemble, which binds resources in doing so.

Another clearer, according to the preamble of claim 1, is known in the art from DE 10 2008 000 610 A1.

Thus, it is the object of the invention to reduce the resource costs, to improve the system capabilities and to render the system more inert to disturbances.

The object is solved by a housing with the features of claim 1. Further, the object is solved by a method of assembling a housing with the features of claim 11.

Advantageous embodiments of the invention are the subject of the dependent claims, the description and the figures. Features, feature combinations, technical effects and advantages described in connection with the housing, the textile machine and/or the clearer also apply to the method of assembling a housing. This also applies the other way around, so that with regard to the disclosure of the individual aspects of the invention, reciprocal reference is or can always be made, particularly independent of the category described.

According to an aspect of the invention, the object is solved by a housing with the features of claim 1.

Therein, a housing of a clearer for a textile machine is particularly configured for guiding a yarn to optically detect characteristics of the yarn, particularly defects or impurities in the yarn. The housing comprises particularly at least a first housing part and a window carrier wherein the window carrier is particularly configured and arranged in a first configuration at the first housing part for a light from a light source to pass at least one of a first window for illuminating a guided yarn. The window carrier is particularly configured and arranged in a first configuration at the first housing part for a light from a light source to pass at least one of a second window to pass the light from the guided yarn to a receiver. The window carrier is detachable from the housing, particularly reversibly detachable from the housing, for transition to a second configuration, particularly for cleaning at least one window. This configuration improves the cleaning of the windows as it allows to disassemble at least the window carrier, particularly as a separate structure and building block of the housing. Therefore, the windows may be cleaned from any dirt that is transferred from the yarns, such as impurities and fiber waste that is transferred via the moving yarn. Detaching the window carrier separately allows to keep the rest of the configuration untouched and therefore speeds up the cleaning process as not the whole process of alignment readjustments have to be repeated. Furthermore, the window carrier may allow to place the respective windows in a beam path such that it allows to reproducibly align the windows with the rest of an optical setup.

Therein, the guiding of a yarn can be implemented such that the yarn passes relative to an illumination spot, an illumination area and/or an illumination volume. In this illumination spot, illumination area and/or illumination volume the yarn may interact with the light emitted from a light source to project a shadow or to reflect light from the yarn to a receiver side. Alternatively or additionally, the yarn, its parts or components thereof may interact with the light and may cause scattering and/or may absorb light of certain wavelengths. All these interactions may be indicative of yarn characteristics, such as impurities, defects, fiber damages etc. In particular, the yarn is guided between two points where it interacts with a yarn-guiding device as described elsewhere herein.

Light yarn interaction allows to optically detect characteristics of the yarn, particularly defects or impurities in the yarn, as described above. To do that, a light source is configured to transmit light via optics to a certain illumination spot, illumination area and/or illumination volume as described elsewhere herein. Additionally, a receiver may comprise optics to transfer light from the illumination spot, the illumination area and/or the illumination volume to a photosensitive detector system (also referred to as photosensitive electronic component). In total, the combination of light source, photosensitive detector and the optics in at least one beam path there between may be referred to as an optical setup.

A first housing part is configured to interact with a window carrier. The window carrier may be configured to carry at least one window which is located on a source side of a gap where the yarn may be guided in. This window may be located closer to the light source against the z-direction of light propagation. The wall in which this window may be placed in at the window carrier may be referred to as an emission side. Alternatively or additionally, the window carrier may carry a second window, wherein the second window is located downstream in z-direction of light propagation and thus closer to the photosensitive detector. The wall of the window carrier wherein the second window may be placed in may be referred to as the receiving side.

The window carrier is configured such that it is detachable, particularly reversibly detachable. Herein, detachable and reversibly detachable refers to the window carrier allows to be removed from the housing without mechanically breaking seals, without ripping apart glued structures or without introducing mechanical damage to the housing, the windows and/or any of the other components of the optical setup and/or the window carrier. Thus, it is possible tc transfer the housing (as well as the clearer as described elsewhere) from a first configuration to a second configuration. A first configuration is a configuration, wherein the clearer can be activated to perform the task of guiding the yarn and particularly of performing an illumination and analysis task thereon. The second configuration is a configuration in which at least one window carried by the window carrier may be removed with the window carrier from the housing. In this configuration the clearer may not be activated as a guiding of the yarn may not be possible. Alternatively or additionally, a moving yarn would introduce dirt to the optical setup of the side (emission side and/or receiving side) where the at least one window is missing as it is removed together with the detachable window carrier.

The window carrier may be configured as a single window carrier, particularly carrying windows from both sides (emission side as well as receiving side). In other embodiments, the window carrier may be configured as several parts which may be detached individually and/or together, particularly in at least two different ways.

According to the invention, a housing comprises housing body as first housing part and a housing cover as second housing part for covering and/or sealing the housing body and can additionally comprise -at least one PCBA, particularly a single PCBA.

The implementation of at least a housing body and at least one other part such as the housing cover or the PCBA allows to simplify the assembly of the housing, particularly in combination with the reduced complexity of optical alignment as described elsewhere herein.

The housing body may be configured to house the optical setup of the emission side (light source and optics) and/or of the receiving side (photosensitive detector and optics). The housing cover may be placed onto or into the housing body to close the housing, particularly sealing the housing from a surrounding.

A PCBA, short for printed circuit board assembly, refers to the combination of a PCB, short for a printed circuit board, and components and electronic accessories. In the housing, the PCBA is placed such that it comprises at least one of the light source or the photosensitive detector. The light source may be a light emitting diode, short LED. The photosensitive detector may be a photodiode that can sense light for transforming it into an electronic signal. At least one of the light source or the photosensitive detector may be placed in plane with the PCB. The combination of a PCB with at least one of photosensitive detector or light source may render it to a PCBA. In embodiments there might be additional electronic components attached to the PCBA. The PCBA may comprise at least one alignment structure that is configured and positioned to allow for an alignment of the PCBA with respect to other optical components described elsewhere herein to assemble the optical setup by placing the PCB onto the respective optical components, particularly placed in the housing body already. The combination and linking of electro-optical components such as light source and photosensitive detector with the PCB may form a PCBA.

According to another aspect, the window carrier may carry at least one window on an emission side and at least one window on a receiving side. A yarn guiding device may be formed by the window carrier to guide the yarn in a position for illumination between the emission side and the receiving side. This allows to transfer both windows into a second configuration for cleaning wherein the window carrier is detached from the rest of the housing, particularly from the housing body. The terms "emission side" and "receiving side" are particularly as described elsewhere herein.

A yarn guiding device is in particular at least one structure, further in particular at least two structures, that is/are configured and positioned to allow guiding the yarn through at least one of an illumination spot, an illumination area and/or an illumination volume. In an exemplary embodiment the yarn guiding device comprises a first contact structure and a second contact structure such that the yarn is moving on a line, particularly a straight line between the first contact structure and the second contact structure. A beam path is particularly implemented such that the yarn crosses it, particularly in an angle, further particularly perpendicular.

According to another aspect, the window carrier may be held by compression between the housing body and the housing cover forming a linkage between the housing body and the housing cover. Thus, the housing may be assembled and the window carrier may be kept in position, particularly without any glue. Therefore, loosening the compression may allow to detach the window carrier from the housing. The compression may be transferred in an exemplary embodiment by at least one screw and/or at least one clip. Tightening the screw allows to increase the compression, loosening the screw allows to reduce the compression. The compression may be transferred by the linkage between the housing body and the housing cover. This allows to detach the window carrier particularly without destroying or damaging a seal material, a glue or any component of the housing.

According to another aspect, at least one of the following may apply:
- no sealing compound or (independent, separate) seal seals the parts contributing to the housing;
- no glue glues between the parts contributing to the housing;
- no electronics are connected to the housing; or
- all electronics are on a at least one PCBA, particularly on a single PCBA.

To ensure that there is no dirt or fiber ingress into the housing, clearers can meet dust protection rating of IP5X. To achieve this level of dust protection, sealings may be used between housing parts. The use of sealings between housing parts requires their production and transport to the assembly position. In case of an elastomer, the elastomer needs to be selected, produced, transported and subsequently assembled. The fitting of an elastomer often comes with issues of form fit: A bit to short and the sealing tends to jump out because of the stretching and tensioning, a bit too long and there is a tendency to buckle following that the surplus material tends to form a buckle which is potentially cut when tightening the housing parts. This may lead to damages in the seal and therefore it may destroy the dust protection rating.

The elastomer seal may experience aging of the material. The material may lose elasticity compared to the date of production. This may lead to potentially sticking issues to a housing part, etc. Also, upon opening of the housing, the seal although visibly in shape might have lost its elasticity which causes a risk after re-tightening of the housing.

Using a sealing compound may further increase the costs of production. During servicing, the sealing compound may need to be broken or opened. Typically, this may be done by heating. During heating, it may have to be ensured, that the storage temperature range of the clearer may not be exceeded. If so, risk of degradation of the housing material (housing body, housing cover) and or internal component alignment exists.

Thus, an embodiment that is free of a sealing compound or (independent, separate) seals for sealing the parts contributing to the housing (housing body and the housed components) from a surrounding allows to improve the sealing capabilities on the one hand. On the other hand, the sealing ratings may be permanently implemented, and implementation may be particularly independent from disassembling the housing and/or from detaching the window carrier. Additionally or alternatively, it allows to assemble or disassemble the housing itself in a resource-saving way. The time for exchanging the window carrier is reduced compared to clearers and/or their housings wherein a seal material or a glue is used.

In embodiments, wherein no electronics are connected to the housing, the housing may be disassembled without having to clip any electronics connection between the PCBA and electronics from the housing. In particular, the electronics such as the PCBA or parts of the active optical components (light source, photosensitive detector) may be taken out of the housing, particularly the housing body, destruction free. Therefore, a reassembly is particularly improved and necessary resources in doing so are reduced.

Using a single PCBA allows to easily place the PCBA, furthermore reducing the demands in alignment. Using particularly a single PCBA allows to detach the PCBA without having to align it with other PCBAs or the optical components attached thereto to be aligned between the different electronical components of independent PCBAs and/or their potential minimal differences in alignment and/or height levels.

According to another aspect eyelets for guiding and stabilizing the yarn in a measurement gap of a clearer may be placed in the window carrier. Alternatively, the eyelets may be placed at the housing body for one part and at the housing cover as the other part.

The term "eyelets" particularly refers to a type of a yarn guiding device that comprises an aperture for guiding a yarn, a C-shape, a U-shape or a V-shape, particularly such that they provide a contact structure for the yarn as described elsewhere herein. They allow to guide the yarn when moving through the measurement gap for optically detecting at least one characteristic of the yarn.

In embodiments wherein the eyelets are placed in the window carrier allow to detach the eyelets with the window carrier. This allows cleaning the eyelets in a cleaning method, particularly in a step or a set of steps wherein the window carrier is detached from the housing to allow cleaning of the windows.

In embodiments, wherein the eyelets are placed at the housing body for one part and at the housing cover as the other part allows to clean the eyelets or to exchange them, when the housing is disassembled.

In an embodiment, air venting holes may be formed either as part of the eyelets or as part of the housing body or as part of the housing cover. In an embodiment, the venting holes may form as a combination of the eyelets and the housing cover or as a combination of the eyelets and the housing body. The venting holes are particularly configured to allow a laminar flow to be established in the area of the moving yarn. The yarn may provide an uneven surface area which may result in a turbulent aur flow when moving. This may result in vibrations disturbing the measurements and/or the movement of the yarn and may further degrade the yarn as fibers can be teared out of the yarn surface. Alternatively or additionally, the air venting holes may allow to compensate for pressure build-up inside the measurement gap due to the moving yarn. This could reduce vibrations as well.

By means of air venting holes to compensate for the static pressure on both sides of the yarn guiding device and/or may generate a laminar air flow on at least one side of the yarn guiding device, at least in the area of the yarn, a significant reduction in the material removal of a yarn monitoring and/or yarn processing unit, in particular a yarn clearer, and at the same time a significant increase in yarn quality can be achieved. Additionally or alternatively, the yarn may follow a particularly constant yarn trajectory between the eyelets, which enables a significant improvement in yarn monitoring and/or yarn processing as well as increased yarn speed, when the air venting holes provide the above-described effects.

An air venting hole particularly refers to a structure, a device or a mean wherein at least or exactly one opening, at least or exactly one passage, or at least or exactly one channel are provided that allows pressure equalization of two areas located on either side of the air venting hole. Alternatively or additionally it allows gas flow or volume flow between these areas (or volumes). The air venting hole can be formed by a single component or by the surfaces or sections of several components. In particular, at least one air venting hole can be formed by at least one combination chosen from the eyelets and the window carrier, the eyelets and the housing cover, the eyelets and the housing body, the window carrier and the housing cover or the window carrier and the housing body. This way, the air venting holes may be adapted to the respective situation and purpose and they may provide a respective air flow properties. In some embodiments, the airflow compensation opening can be completely located within a component and/or formed entirely by a component, whereby the component also particularly provides the yarn guiding device, particularly a yarn guiding opening. The air venting holes may particularly be located adjacent to the yarn guiding device, further particularly the yarn guiding opening.

In an embodiment of the yarn guiding device, the air venting holes particularly break through the yarn guiding device, or the yarn guiding device's outer wall, in the yarn moving direction. Alternatively or additionally it completely comprised therein. Alternatively or additionally, it is spaced apart from and/or adjacent to the yarn guiding device, particularly the yarn guiding opening, which allows for particularly efficient pressure equalization and/or laminar airflow in the area of the yarn movement. Particularly, the yarn guiding device, further particularly the yarn guiding opening and the air venting hole(s) run at least in the area of one side of the yarn guiding device and further particularly completely separated from each other. In particular, the air venting hole is particularly formed and/or arranged in such a way that the yarn cannot enter the air venting hole. In particular, a design of the yarn guiding device is preferred, in which the air venting hole is located on one side of the yarn guiding device, particularly the yarn guiding opening, facing away from a yarn intake opening, preferably in a front view of the yarn guiding device below the U-, C- or V-shaped yarn guiding area where the two yarn guiding sections (the two shanks of the U-, C-, or V-shaped yarn guiding area) converge. Alternatively or additionally, they are placed centrally in relation to the yarn guiding device, particularly the yarn guiding opening.

According to the invention, a (reversed) gravity seal is formed by the housing cover sunken into the housing body to be oriented such that the housing cover forms the bottom of the housing. This allows to implement a dust protection rating of IP5X or IPX6 as they are established on date of filing or date of priority.

A (reversed) gravity seal is a seal that does not rely on an independent, separate seal material, such as an O-ring or an elastomer that is placed in a respective recess in the respective housing body and/or in the housing cover. Instead, a sealing that is a combination between form consistent part design and a use of gravity as an effect counteracting the tendency of dust to penetrate form consistent parts of at least two of a housing body, a housing cover or of a window carrier.

The housing particularly comprises a bottom and a top, wherein the bottom is formed by a housing cover which is particularly designed to be sunken into the housing body as a form consistent part. The bottom is thus particularly to be positioned in a direction of gravity lower than a top. Therefore, the housing body particularly forms the top component.

According to another aspect, a meandering seal may be formed such that at least a first wall and a second wall are positioned and configured such that they run at least together at a seam to be sealed, particularly parallel to each other, wherein both walls at least provide one angular turn to force dust entering the seal and migrating between the at least first and second wall to change direction at least once, particularly several times. This forces the dust, if entering the seal, to migrate on long migration paths and to even change direction. Thus, the friction in the seal increases for the dust over time and supports the sealing effect.

In an embodiment, particularly where a window carrier can be sealed where it contacts a housing, the dust would have to move first sideways to the back of the clearer and/or of the housing body, then horizontally to the side of the housing body, followed by a forwards movement, followed by again a horizontal movement, then followed by a repeated movement to the back of the housing after which another horizontal stretch needs to be passed followed by a third movement to the back succeeded by fourth horizontal movement and a fourth movement to the back after which a reversed sideways stretch is to be passed and then a final fifth movement to the back. In this and alternative embodiments several angles as well as reversed motion directions can be provided. These angles and reversed motions do not have to be full U-turns, but can be direction changes, particularly deviating from the direction of the seam in at least one angle, particularly in a U-turn. Alternatively, several different angles can be chosen and also several meandering lengths of the path provided for the dust to pass. The particular scheme may be provided such that it achieves the desired dust protection rating. The embodiment of the double meandering seal can achieve IP6x dust protection rating as it is defined on the date of filing or the date of priority.

According to another aspect, a meandering sealing may be formed such that from a seam of the housing cover sunken into the housing body a wall protrudes upwards into the housing. The sealing may be a combination between form consistent part design and a meandering sealing. The sealing may meander around the external circumference of at least one of the housing body, the housing cover or the window carrier. To penetrate the housing cover seal, fluff, dust, dirt and fiber debris as well as fibers may have to move up between the housing body side wall and the housing cover, then horizontally between housing body and housing cover.

Alternatively or additionally, a double meandering sealing may be formed such that from a seam of the housing cover sunken into the housing body a wall protrudes upwards into the housing up to a first step, where the wall recesses in a direction from a sidewall of the housing body and protrudes upwards up to at least a second step. To penetrate the housing cover seal, fluff, dust, dirt and fiber debris as well as fibers may have to move up between the housing body side wall and the housing cover, then horizontally between housing body and housing cover, thereafter it may have to move up again.

Alternatively or additionally, a double meandering sealing may be formed such that from a seam of the housing cover sunken into the housing body a wall protrudes upwards into the housing up to a first step, where the wall recesses in a direction from a sidewall of the housing body and protrudes upwards up to a second step and wherein a second wall is placed next to the first wall forming a valley between them. To penetrate the housing cover seal, fluff, dust, dirt and fiber debris as well as fibers may have to move up between the housing body side wall and the housing cover, then horizontally between housing body and housing cover, thereafter it may have to move up again. Thereafter the particles, fiber debris and fibers may have to pass through the valley and then "cross" another barrier in form of the second wall. All the paths the debris, dirt, dust, fibers and other particles (which will be addressed by the collective term dust) will have to take are between form consistent parts, so parts whose contours are aligned and structured to match their counterparts such as the housing body where it aligns with the housing cover. Therefore, the "paths" are highly friction affiliated paths, likely to block the dust at some point.

According to another aspect, a meandering sealing may be formed such that from a seam of the housing cover sunken into the housing body a wall protrudes upwards into the housing at a position where the window carrier engages with the housing cover. In respective embodiments the advantages as described with respect to the meandering seal between the housing body and the housing cover apply. For matter of readability and conciseness, recasting thereof is avoided.

Alternatively or additionally, a meandering sealing may be formed such that from a seam of the housing body at a position where the window carrier engages with the housing body a wall protrudes downwards into the window carrier. In respective embodiments the advantages as described with respect to the meandering seal between the housing body and the housing cover apply. For matter of readability and conciseness, recasting thereof is avoided.

Alternatively or additionally, a double meandering sealing may be formed such that from a seam of the housing cover sunken into the housing body a wall protrudes upwards into the housing at a position where the window carrier engages with the housing cover, up to a first step, where the wall recesses in a direction from a sidewall of the housing body and protrudes upwards up to at least a second step. In respective embodiments the advantages as described with respect to the meandering seal between the housing body and the housing cover apply. For matter of readability and conciseness, recasting thereof is avoided.

Alternatively or additionally, a double meandering sealing may be formed such that from a seam of the housing body at a position where the window carrier engages with the housing body a wall protrudes downwards into the housing, down to a first step, where the wall recesses in a direction from a sidewall of the housing body and protrudes downwards down to at least a second step. In respective embodiments the advantages as described with respect to the meandering seal between the housing body and the housing cover apply. For matter of readability and conciseness, recasting thereof is avoided.

Alternatively or additionally, a double meandering sealing may be formed such that from a seam of the housing cover sunken into the housing body a first wall protrudes upwards into the housing at a position where the window carrier engages with the housing cover, up to a first step, where the wall recesses in a direction from a sidewall of the housing body and protrudes upwards up to a second step and wherein a second wall is placed next to the first wall forming a valley between them. In respective embodiments the advantages as described with respect to the meandering seal between the housing body and the housing cover apply. For matter of readability and conciseness, recasting thereof is avoided.

Alternatively or additionally, a double meandering sealing may be formed such that from a seam of the housing body at a position where the window carrier engages with the housing body a first wall protrudes downwards into the housing, down to a first step, where the wall recesses in a direction from a sidewall of the housing body and protrudes downwards down to at least a second step and wherein a second wall is placed next to the first wall forming a valley between them. In respective embodiments the advantages as described with respect to the meandering seal between the housing body and the housing cover apply. For matter of readability and conciseness, recasting thereof is avoided.

According to another aspect, the housing may comprise a button with a light guide for a signaling light source, particularly a signaling LED. This allows to implement indicating the state of the clearer to the operator. These indicating lights from light sources may be implemented by one or multiple LEDs. The LEDs can have single color or consist of multiple colors. A typical intuitive color scheme when running may be green for ok and red for a fault (traffic light colors). Besides these states there may be many other states of which the operator is to be informed: length of yarn to be removed, clearer blocked, incorrect yarn parameters, communication faulty etc. The light sources may emit coded light flashes in certain flash frequencies of exchanging on-off states of the light source. An exemplary method may be to use blinking schemes of the LEDs used for indication. The blinking period and blinking scheme may indicate the state. The blinking scheme is typically extended to all of the LEDs available for indication use. In a way conveying morse signs to the operator.

In an exemplary embodiment a signaling RGB (red green blue) LED is proposed by which the most important states can be indicated by intuitive color coding. With the RGB LED traffic light signaling as well as other states can be implemented. With the inclusion of a single timer on the signaling RGB LED the number of states can be doubled, moreover closely related states or problems can be indicated by color + non blinking and color + blinking. For instance, three different colors to indicate the amount of yarn that is to be removed (short, middle, long). An alternative exemplary embodiment is to choose a color in combination with a timing sequence to indicate the amount of yarn that is to be removed. In these signaling schemes, either a positive or a negative regime can be adhered to. A positive scheme may indicate an amount of yarn that is to be removed, a negative scheme may indicate that yarn is to be removed until all the yarn that is to be removed has been removed. In a positive scheme, "short blinking" may indicate a "short fault" with "little yarn to be removed", a "middle period of blinking" might indicate a "middle fault" indicating that "several meters of yarn have to be removed", whereas a "long period of blinking" indicates a "long fault" for "considerable yarn needs to be removed (i.e. 10m or more)". In another embodiment adhering to a negative regime, the signaling LED keeps indicating yarn to be removed for as long as there is still yarn to be removed.

The signaling LED can be linked to a light guide. The shape of the light guide is in particular such that it emits an elliptical illumination pattern: the side wards emission angles to the adjacent spindles of a textile machine may be considerably larger compared to the emission angles in up (top) and down (bottom) direction. This emission pattern may give better visibility to the operator from far and may reduce power consumption or LED loading time, potentially influencing the blinking frequency, particularly due to improved efficiency. Further to the shape of the dome at the top of the light guide, ellipsoidal Fresnel lenses or other structures impressed to the light emitting surface of the light guide can be incorporated in addition to improve light distribution into the desired direction. Another feature for improvement of the light guide efficiency may be to adapt the surface of the light guide at the bottom where the light of the LED may be coupled into according to the emission profile of the signaling RGB LED and the desired light distribution, i.e., typically domed as well, alternatively a conus shape.

Besides indicating the state of the clearer to the operator, in an alternative exemplary embodiment it is possible to receive feedback from the operator, for instance an acknowledgement or a confirmation that a certain corrective process or handling has been completed. To get the feedback from the operator a button can be integrated into the housing of the clearer. The button can be an additional component that is to be placed onto the housing. In the housing provisions can be made for the button, the parts can be produced, assembled and checked according to the respective function of the button.

Another exemplary embodiment is to integrate the button function together with the indicating light guide. This reduces production costs. An alternative or additional embodiment comprises a mechanical solution wherein the light guide is movable e.g., pushed down by the operator if so required and retracting on its own upon release. This provides a simple to implement and robust alternative. A mechanical solution may be independent of the means by which the button is pressed. As an added feature, a mechanical solution may be able to give tactile feedback to the person pressing it.

An alternative exemplary embodiment to a mechanical button may be an all-optical solution. In an all-optical solution, wherein an additional photodiode may be used with which the variance in brightness of the returned or back-reflected light of the light guide upon activation or blocking or reflection by the operator's hand of the light guide can be measured. Respective embodiments can avoid moving parts.

For embodiments with a mechanical button, the light guide may be configured to move down when being pressed by the operator and to retract on its own upon release. The spring-loaded action from the light guide may be implemented by a sealing. The sealing may have a sealing dome, which can be compressed when pressed and which can return to its original shape when released. The tactile feedback may be implemented by activating a micro-switch at the end of the stroke of the light guide. To potentially avoid the micro-switch of being over-pressed by the operator, a mechanical stopper may be integrated into the PCBA to which the tactile switch is particularly assembled. By integration of the stopper on the same PCBA as the tactile switch, all tolerances can be controlled during PCBA manufacturing with low cost and high accuracy. Tolerances during assembly or other tolerance stack ups can be avoided.

In an exemplary embodiment, a flange may be integrated underneath the button and/or underneath the light guide that can (significantly) be wider compared to the opening in the housing body where button seals can be placed. The light guide and/or button can be assembled with its domed seal (therefore also referred to as seal dome) from the inside of the housing prior to assembly of the PCBA. Through the integrated flange, the button to the outside may be constrained by the housing and to the inside by the PCBA (after its assembly). The flange may avoid that the button can be removed from the housing, for instance by a fingernail gripping in the seam between housing and button.

According to an independent aspect, a clearer may comprise a housing, particularly as described throughout this description. A clearer may be used in before or after a yarn guiding roller before the yarn is transferred via a changing device transferring the yarn to a spindle. The clearer (also to be called yarn clearer) may be used for cleaning the yarn from particular impurities in its surface structure or from fibers which are respectively pointing outwards from the yarn. Features, feature combinations, technical effects and advantages described in connection with the housing, the textile machine and/or any of the methods described elsewhere herein also apply to the clearer, irrespective of the category (method, use, device and system) described or claimed. This also applies the other way around, so that with regard to the disclosure of the individual aspects of the invention, reciprocal reference is or can always be made, particularly independent of the category (method, use, device and system) described and/or claimed.

According to an independent aspect, a textile machine may comprise a clearer, particularly as described throughout this description. Features, feature combinations, technical effects and advantageous described in connection with the housing, the clearer and/or any of the methods described elsewhere herein also apply to the textile machine, irrespective of the category (method, use, device and system) described or claimed. This also applies the other way around, so that with regard to the disclosure of the individual aspects of the invention, reciprocal reference is or can always be made, particularly independent of the category (method, use, device and system) described and/or claimed.

According to an independent aspect, a method of assembling a housing, particularly as described throughout this description, or a clearer, particularly as described throughout this description, may comprise a step of placing at least one optical component, particularly all optical components, further particularly the optical parts for providing illumination from a source to a yarn (without the windows), further in particular from a yarn to photosensitive detector, in the housing body. The method may comprise a step of placing the PCBA (particularly the PCB to form a PCBA together with the electrooptical components) in the housing body, particularly after placing at least one optical component, further particularly independent thereof. The method may comprise a step of connecting the optical components and the PCBA. Features, feature combinations, technical effects and advantageous described in connection with the housing, the clearer and/or the textile machine described elsewhere herein also apply to any of the methods described elsewhere herein, irrespective of the category (method, use, device and system) described or claimed. This also applies the other way around, so that with regard to the disclosure of the individual aspects of the invention, reciprocal reference is or can always be made, particularly independent of the category (method, use, device and system) described and/or claimed.

According to another aspect no optical (fine) alignment is performed on the (active electro-)optical components to be linked with the PCBA except for mounting the (active electro-)optical components to the same plane on the PCBA (giving a course alignment). This simplifies the alignment process and improves the handling of the assembly of the housing with an optical setup therein. This further reduces the resources necessary for alignment and assembly.

Here, the term "fine alignment" particularly refers to an individual alignment of the individual active electrooptical components such as the light source on the emission side or the photosensitive detector on the receiving side. Alternatively or additionally, no individual alignment of individual optical components in the optical setup has to be performed upon placing the optical components in the housing, particularly before contacting the PCBA. The term "course alignment" particularly refers to the alignment of the active and/or passive optical components being given by placing them in respective pockets of the housing. The course alignment is particularly given in that the PCBA (particularly as PCB) is placed in the housing, particularly after placing the optical components therein. Thus, it is not mandatory to assemble an optical system connected to the PCBA and then housing this combination, but instead the optical components and the optical setup is placed in the housing, then the PCBA is added to the housing and then the respective electrical connections to the active optical components of the optical setup can be established. The optical components of the setup are particularly cased in casings which fit into pockets in the housing that align the optical setup when placed therein.

According to another aspect, a window carrier may be placed in the housing body. Therefore, the window carrier may arrange at least one window into the beam path of the optical setup to protect the optical setup from dust entering the optical path and the housing deteriorating the quality of the measurements to be performed.

The window carrier may be placed particularly independent of any other component into the housing body. This allows to reversibly detach the window carrier from the housing body.

According to another aspect, alignment structures may align the PCBA with the optical components. Those alignment structures may be pins, protrusions or recesses that engage with through-holes, holes, recesses, or any other given structure that is capable of fixing a position of PCBA in a certain plane and height position in the housing. The housing body may comprise holding structures such as ribs that place the PCBA in a certain height in the housing body and at certain positions in the housing body in a plane with respect to the (side) walls of the housing body.

According to another aspect, the housing cover may be sunk into the housing body, particularly forming the meandering sealing, further particularly the double meandering sealing, particularly without placing an independent (separate) seal. This avoids individual, separate seals which might deteriorate over time, are complicated to manufacture in the necessary tolerances, which might lead to misplacement of the housing body relative to the housing cover and other disadvantages already explained elsewhere herein. The respective dust protection rating IP6X is possible, at least IP5X can be provided, both as defined on date of filing or date of priority.

In the following, embodiments of the invention are described in more detail with reference to figures, showing schematically and exemplarily:
- Fig. 1: an embodiment of a clearer;
- Fig. 2: an embodiment of a housing of a clearer in exploded view;
- Fig. 3: an embodiment of a mirror holder;
- Fig. 4: a schematic diagram of an embodiment of an optical setup;
- Fig. 5: a schematic diagram of an embodiment of an optical setup;
- Fig. 6: a schematic diagram of an embodiment of an optical setup;
- Fig. 7: a schematic representation of an embodiment of an aperture with passing yarn;
- Fig. 8: a schematic ray diagram of an embodiment of an optical setup;
- Fig. 9: a schematic ray diagram of an embodiment of an optical setup;
- Fig. 10A: a top perspective view of an embodiment of a button;
- Fig. 10B: a top perspective view of an embodiment of a button with a seal;
- Fig. 10C: a bottom perspective view of an embodiment of a button with a seal;
- Fig. 10D: a partial cut view of an embodiment of a seal for a button;
- Fig. 10E: a button with seal placed at a housing;
- Fig. 11: a cut view through a plane parallel to the yarn propagation direction through a housing;
- Fig. 12A: a cut view through a first plane perpendicular to the yarn propagation direction through a housing showing a selected part thereof;
- Fig. 12B: a cut view through a second plane perpendicular to the yarn propagation direction through a housing showing a selected part thereof;
- Fig. 13A: a cut view through a first plane perpendicular to the yarn propagation direction through a housing shown in full, partially assembled;
- Fig. 13B: an embodiment of a window carrier with an embodiment of a lighthouse;
- Fig. 13C: an embodiment of a lighthouse; and
- Fig. 13D: a cut view through a first plane perpendicular to the yarn propagation direction through a housing shown in full, partially assembled with optical setup placed therein;

The same reference signs are used for elements and structures exhibiting the same effect and/or which are of the same type.

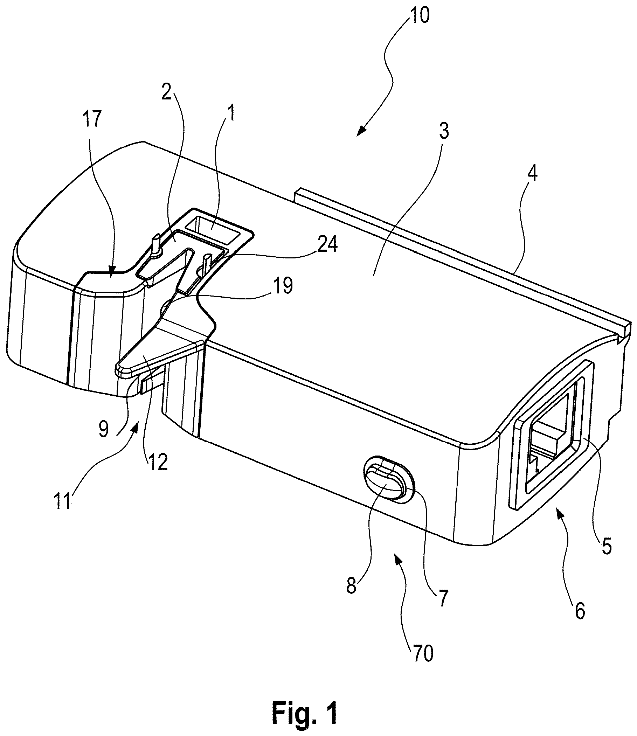

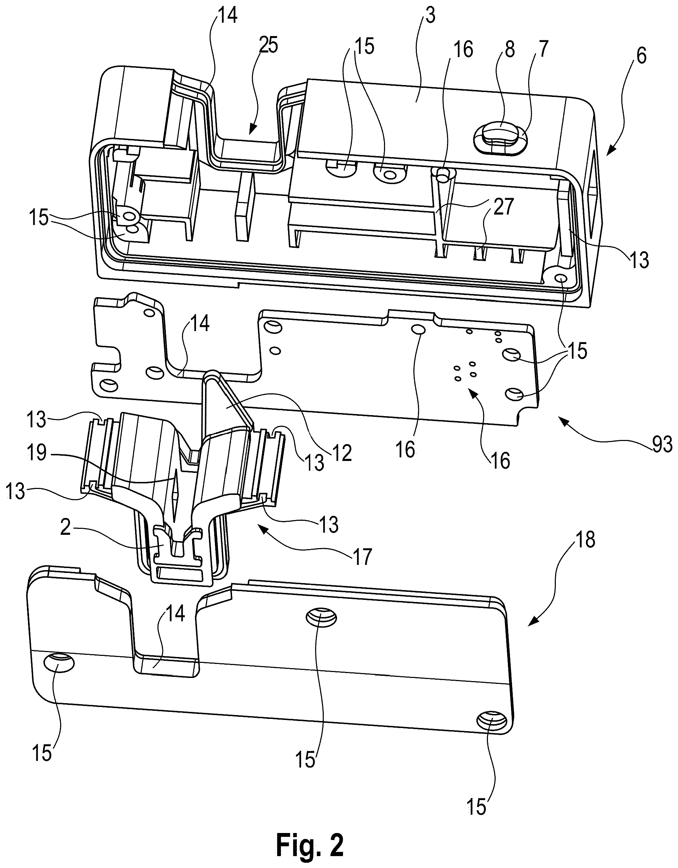

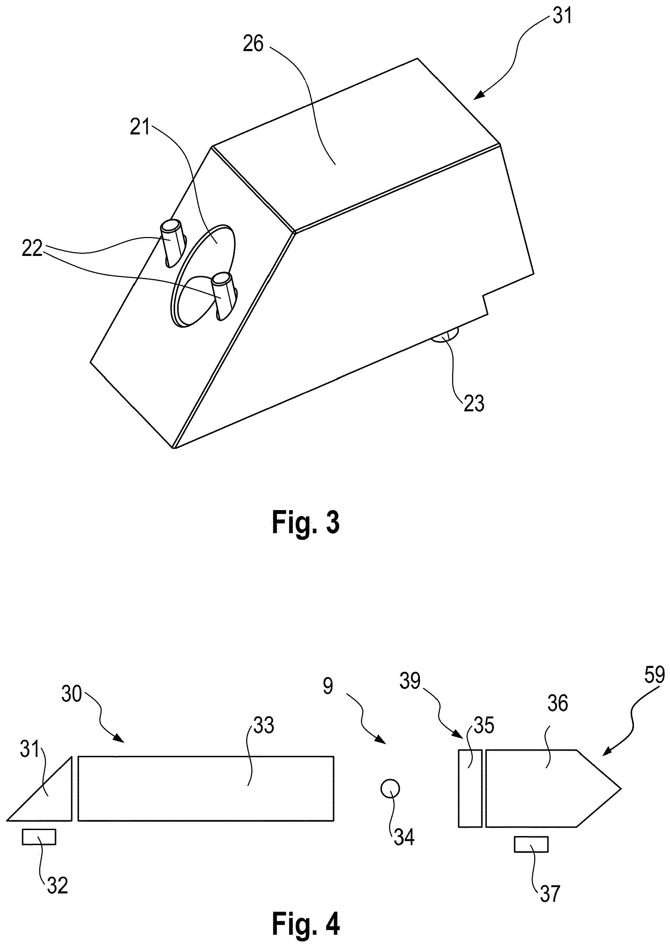

Fig. 1 shows an embodiment of a clearer 10, whose optical, mechanical and electrical components, their production process, the assembly processes as well as the service processes are addressed in the following. The clearer 10 can be used to measure the thickness of a yarn 34 (see Fig. 4). A through-light shadow-projection setup may be used in some embodiments. For correct measurements of the diameter of the yarn 34 an even, non-divergent illumination profile without any vignetting can be provided. The clearer 10 combines an illumination projector 30 with an illumination receiving system 39, both as described in detail for different embodiments with respect to Figs. 3 to 6. The clearer 10 provides a housing that is described in detail with respect to Fig. 2. The clearer 10 is configured to be placed in a textile machine and may be configured for guiding a yarn 34 to optically detect characteristics in the yarn 34. To do so, the clearer 10 may be placed after a yarn 34 was made in the textile machine and before the yarn 34 is transferred to a spindle for transportation or storage.

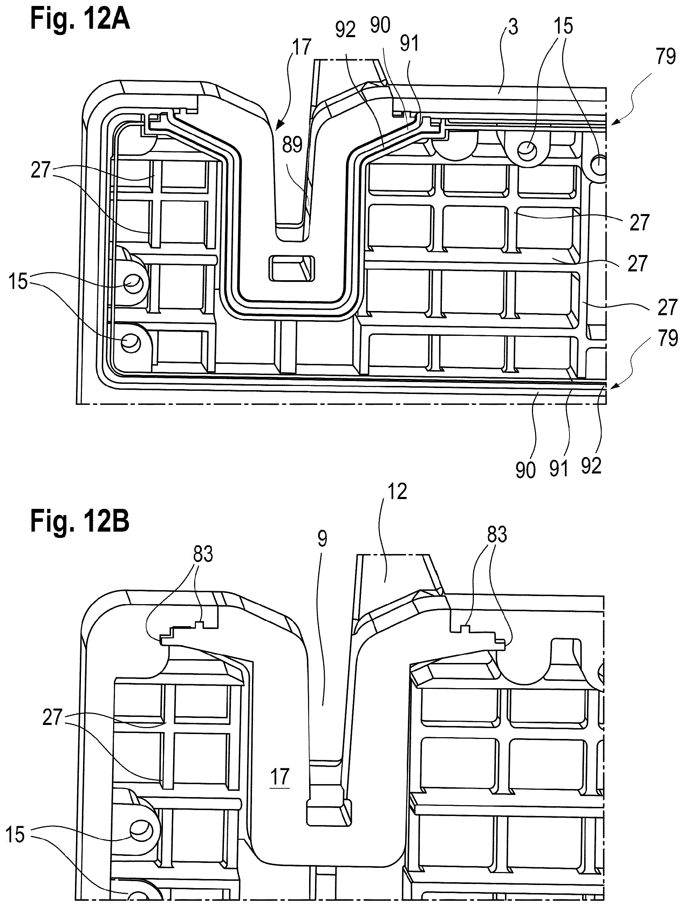

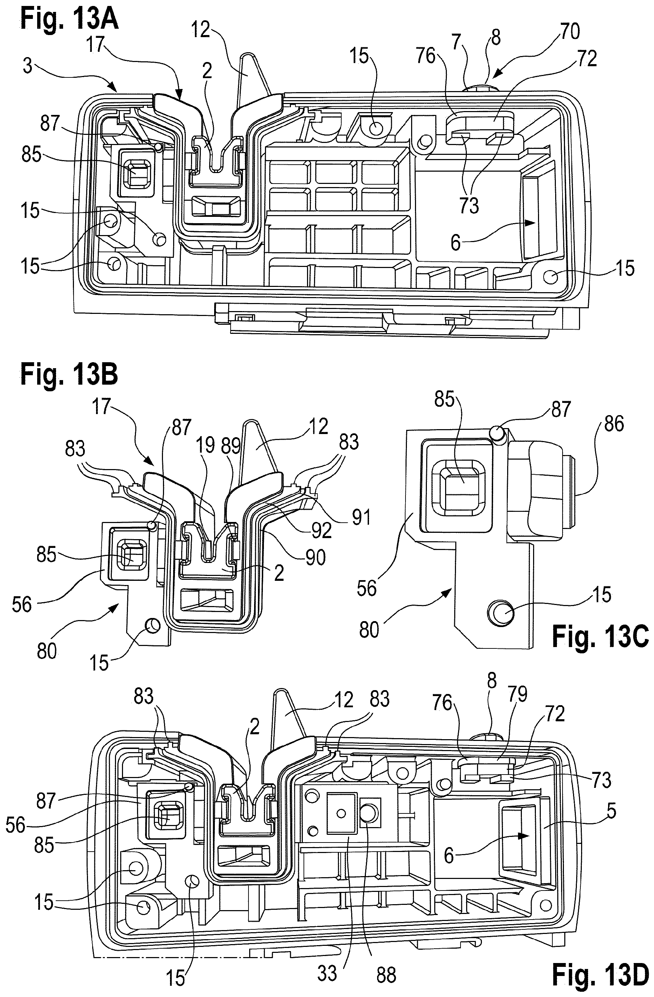

The clearer 10 comprises a housing body 3 that can be mounted to a textile machine via a mounting element 4. A yarn 34, which is not shown in Fig. 1, can pass a yarn guiding device **11** for yarn guiding. The yarn guiding device **11** can comprise at least two eyelets 2 which provide contact points for the yarn 34 to move in a measurement gap 9 of the window carrier 17, between a first window 19 on an emission side (where the illumination projector 30 projects light in a z-direction of propagation through the measurement gap) and a second window 89 (here not visible for reasons of perspective; please refer to Fig. 12A and 13B). Particularly between these two windows, light propagates to interact with the yarn 34. An air venting hole 1 can be provided to reduce vibrations from air flow and pressure build-up in the yarn guiding device 11, avoiding transfer of vibrations to the optical components to improve the measurement quality. The air venting hole 2 may be provided, as shown in Fig. 1, in the window carrier 17 alone, but may in other embodiments be formed by a combination of at least two of the window carrier 17, the eyelets 2, the housing body 3 or the housing cover 18. A yarn exit guide 12 may facilitate guidance of the yarn into the measurement gap 9 during automatic yarn placement. In addition, yarn exit guide 12 may prevent the yarn 34 from slipping out of the measurement gap 9 in case it is transferred to a transportation or storing spindle thereafter and changing the pointing therefore rapidly.

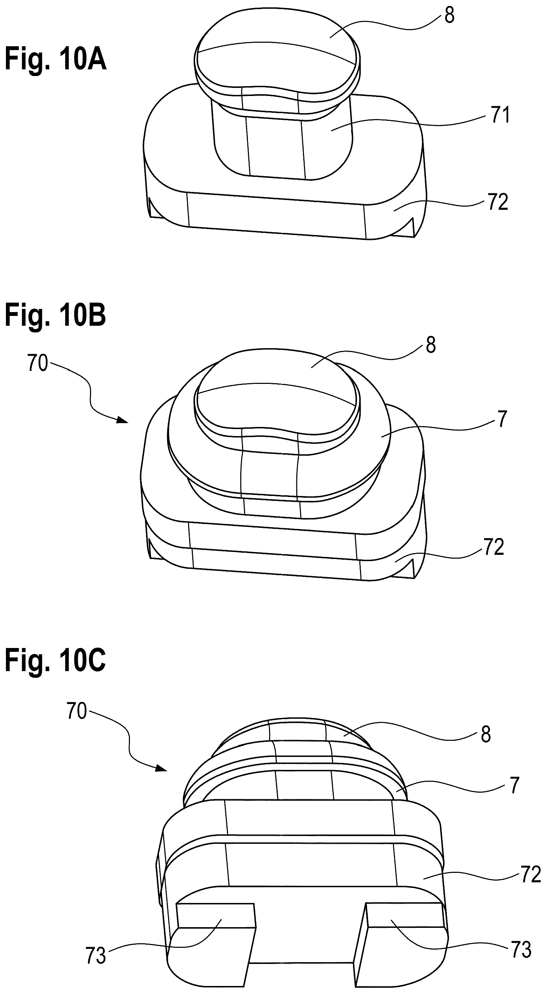

The clearer 10 can indicate certain measurement results via a button 8 with light guide 70 wherein the button 8 is placed in a seal dome 7 as described in great detail with respect to Figs. 10A to 10E. The clearer 10 may comprise a connector plug 6 (or a respective data transfer cable or wireless transmission device in other embodiments not shown here) in the housing body 3 which allows to transfer data and results from the measurements to a computer for example. The connector plug 6 may comprise a connector sealing 5 to avoid dust from entering the housing.

Fig. 2 shows a housing of a clearer 10 in an exploded view. The housing may comprise at least a first housing part and a window carrier 17. The window carrier 17 is configured and arranged in a first configuration at the first housing part for a light from a light source 32 (see Fig. 4 to 6 for details) to pass at least one of a first window 89 for illuminating a guided yarn 34 or a second window 19 to pass the light from the guided yarn 34 to a receiver (see Fig. 4 to 6 for details) as described in detail elsewhere herein. The window carrier 17 can be configured to be detachable from the housing, particularly reversibly detachable from the housing, for transition to a second configuration, particularly for cleaning at least one window 19, 89.

The housing may comprise a housing body 3 as first housing part and at least one PCBA 93. In some embodiments, as one is shown here, particularly a single PCBA 93 may be provided. Alternatively or additionally, a housing cover 18 may be provided as second housing part for covering and/or for sealing the housing body 3.

The window carrier 17 may carry at least one window 89 on an emission side and at least one window 19 on a receiving side. As already described with respect to Fig. 1, a yarn guiding device 11 may be formed by the window carrier 17 to guide the yarn 34 in a position for illumination between the emission side and the receiving side, so through the measurement gap 9.

In the exemplary embodiment shown, the clearer 10 housing consist of 3 parts: the housing body 3, carrying the single PCBA 93 and the detachable part carrying the windows 19, 89 (wherein the detachable part is therefore referred to as window carrier 17), the housing cover 18, covering and sealing the housing body 3 as well as the detachable window carrier 17. The housing cover 18 is particularly attached to the housing body 3 by screws. The window carrier 17 may be held by compression as well as by form linkage between the housing body 3 and the housing cover 18.

As shown in the exploded view in Fig. 2, particularly no individual, separate sealing compounds or seals, e.g. made out of elastomers, silicons and or rubbers, are used to seal the parts contributing to the housing, other than a seal dome 7 for an optional button 8 and a connector seal 5 for the connector plug 6. Further in particular, no glue is used between the parts. Further in particular, no electronics are connected to the housing, particularly all electronics are provided on a single PCBA 93. All internal connections can preferably be made by using screws (not shown here) placed in screw holes 15.

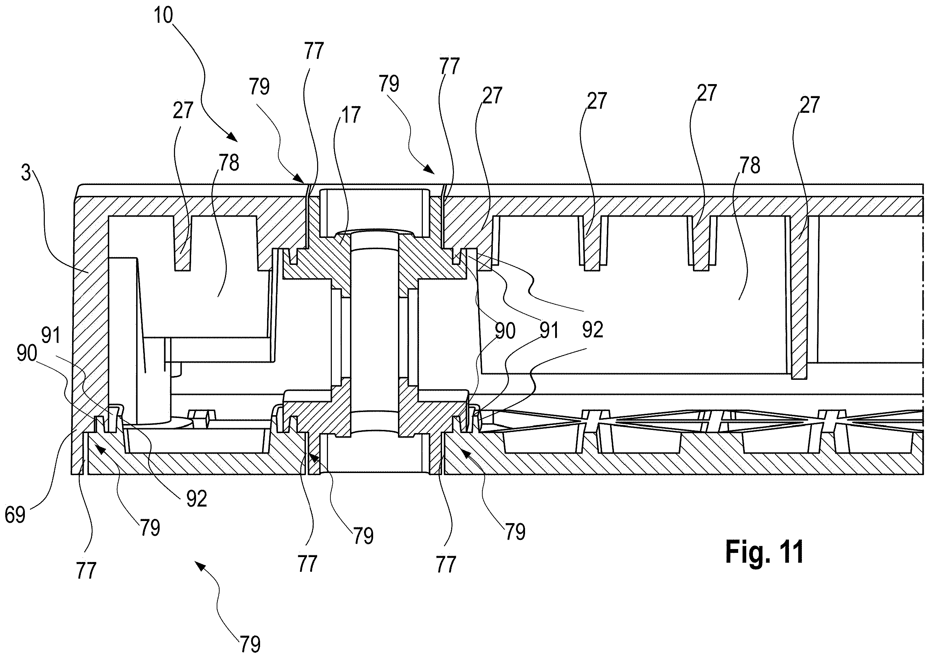

As a result, the whole housing including all the internal parts can be efficiently assembled with ease. To align all the components, ribs 27 allow to place the components in the right height and relative position inside the housing body 3, referred to as pockets 78 (see Fig. 11). Therefore, no special care may have to be taken during alignment, nor special thermal treatments may be needed for stress relief after assembly. As a result, the window carrier 17 can be easily exchanged by just removing and retightening a few screws. No re-calibration, no stress relief process may be required. Depending on the housing design, the detachable window carrier 17 can also carry the eyelets 2 for guiding and stabilizing the yarn 34 in the measurement gap 9 (see Fig. 1) of the clearer 10. An alternative design may be to integrate the eyelets 2 into the housing body 3 for one part and into the housing cover 18 as the other part. Alignment structures 16 may provide the respective support to pre-align all components to be placed inside the housing body 3 upon assembly.

The window carrier 17 comprises the windows 19, 89 as described elsewhere. The window carrier 17 may be placed in recesses 25 contacting a contact surface 14 for the window carrier 17 on the housing body 3 and similar contact surfaces 14 on the PCBA 93 and the housing cover 18. The window carrier 17 may interact with the housing body 3 via connector pins 13 and/or sleeves that form respective orthogonal sealing and locking features 83 as shown in Fig. 12B showing a cut view through a plane through the connector pins 13. It is understood that the connector pins 13 can be implemented pointwise as individual pins or pockets for location and or sleeves with a longitudinal shape covering location in combination with sealing.

Fig. 3 shows an illumination projector 30 with integrated mirror housing 31 functioning as a mirror holder for an exemplary embodiment of a waveguide 33 as shown in Fig. 4. Therein, an exemplary illumination projector 30 design shows an 45° angle for second surface mirror attachment, wherein the offset correction for the thickness of the second surface mirror as mirror 21 as well as the heat stacks 22 are shown in Fig. 3, to which the stamped mirror foil as mirror 21 can be placed and wherein heat stacks 22 can subsequently be molten to secure the mirror 21 in place.

As shown in Fig. 4, the LED as light source 32 is mounted in plane together with a receiving photodiode as photosensitive electronical component 37 on the PCB to form a PCBA 93, particularly being used for signal processing. The illumination projector 30 can have the light to be emitted by the light source 32, particularly in form of an LED, whereby the light source 32 is particularly mounted directly to the surface of the signal processing PCB, forming the PCBA 93, to particularly having a 90° angle of the illumination projection. The 90° illumination angle of the illumination projector 30 can be achieved by using a simple 45° mirror 21 added at the side of the light source 32, particularly the LED, to the waveguide 33, the waveguide 33 particularly being in form of a tube 26. A potential embodiment on mounting a mirror 21 together with the tube 26 as a single integrated component is shown in Fig. 3.

The 45° mirror 21 can be shaped and function as a prism (not shown), particularly using total reflection, or by adding a mirror coating at the 45° surface (as suggested in Fig. 4), by means of a simple first surface mirror (not shown) directly mounted to the illumination projector's 30 structure (not shown), or even simpler by means of a second surface mirror 21.

An exemplary low cost means of integrating a mirror surface to the illumination projector 30 can be achieved by using a second surface mirror foil. The mirror foil can be particularly easily produced by stamping large sheets of abundantly available commercial mirror foils to the required size, particularly so as the surface mirror coating may be mechanically protected. The base materials used for these foils can be one of PE, PP or PMMA, all of which having low surface tensions compared to glass or aluminum. The low surface tension may be an inherent protection against vapor condensation. If so required, the already low surface tension can be further reduced by additional surface coatings on sheet basis. The thickness of the foils can be selected such, that the offset in the projection of the light (light bundle) of the second surface is negligible in practical terms, for instance by selecting secondary mirror foils with a thickness ranging from 0.05 to 0.15 mm. In case thicker foils are used, the offset by the secondary surface can be easily accommodated by correct optical arrangement of the actual waveguide 33, particularly in form of a projection tube 26 of the illumination projector's 30 structure.

The secondary surface mirror can be secured to the illumination projector 30 using for instance glue. Direct gluing of the mirror 21 with particularly a low surface tension second surface to the illumination projector 30 can be avoided. A mechanical means of securing the mirror is for instance by means of a pocket in which the mirror 21 can be slit into following which the pocket being sealed off afterwards with i.e., glue. This is particularly done in production as additional sliders may be used during manufacturing of the illumination projector 30, but also the sliding of the mirror 21 into and the sealing of the pocket may be done. Moreover, the mirror 21 might get scratched during sliding into this pocket. Another alternative method may be applied by creating a box into which the mirror 21 can be placed followed by placing and/or pressing a cover into the box to be able to secure the mirror 21. In those embodiments, box as well as cover are particularly to be provided. This is further simplified (further saving resources) by a method of heat staking: during injection molding heat stacks 22 can be made to the sidewall of the waveguide 33, particularly as a projection tube 26. As the secondary mirror foil can be stamped, holes can be made at the position of the heat stacks 22. During assembly the mirror 21 can be placed over the heat stacks 22 of the illumination projector 30. In the following the heat stacks 22 can be molten down to the lining of the mirror foil. The result is a low-cost production process that can have a very high yield, that is particularly clean and yields a secure mirror attachment regardless of environmental conditions and thermal cycling.

To reduce sensitivity of the illumination receiving system 39 to environmental light in the measurement gap 9 of the yarn 34 and to reduce influence of back reflected stray light from the receiving optics, the exit aperture of the waveguide 33, particularly the projector tube, can be light absorbing (i.e. black). Furthermore, the end material may be non-specular and may have a shape that directs any light reflected off its surface away from the illumination receiving system 39 (which may be referred to as the receiver).

Fig. 4 shows a schematic diagram of an exemplary embodiment of an optical setup. Therein, an illumination projector 30 is shown which is configured for a shadow projection from a yarn 34 in a textile machine. Alternatively or additionally, the illumination projector 30 may be configured for illuminating a yarn 34 for light to be reflected from the yarn 34. The illumination projector 30 may be independently implemented from an illumination receiving setup 39 as shown elsewhere herein.

The illumination projector 30 particularly comprises at least one light source 32, particularly a light emitting diode or in short an LED. The illumination projector 30 particularly comprises at least one waveguide 33 and particularly a yarn guiding device 11. The light source 32 is particularly placed such that the emitted light enters the waveguide 33. The waveguide 33 is placed such that the light exiting the waveguide 33 particularly forms an area-source like emission on its exit side. The yarn guiding device 11 is particularly configured and positioned relative to the area-source like emission on the waveguide's 33 exit side to project a shadow from the yarn 34 to a receiving side or to reflect light from the yarn 34 to a receiving side. At the receiving side there may be one of different embodiments of an illumination receiving setup 39 as described elsewhere herein.

The waveguide 33 comprises in particular a tube, further in particular, the tube is a light-homologation tube. The purpose of the light-homologation tube is to destroy any pattern or image carried in the propagating light beam e.g., an emitter pattern of an LED. The cross section of the tube contained in waveguide 33 can be of a round, elliptical, rectangular or squared shape. In longitudinal direction, the tube may be straight or conical.

The cross section and the width of the waveguide 33 can be designed according to the required width of the light bundle and the height of the waveguide 33 can be designed according to the height requirement of the light bundle. In particular, the width of the waveguide 33 and the height of the waveguide 33 are individually and independently shaped according to the measurement requirements, wherein the width of the light bundle is adjusted to the width resolution e.g., of the yarn defects to be measured. They may correspond to the minimum length of the yarn defects to be detected. The height of the light bundle is particularly adjusted to the maximum size of the yarn defect to be measured.

The waveguide 33 may comprise a hollow core with an inner surface being diffusive and/or non-specular. This way a maximum of light may be propagated through the waveguide in form of a tube, particularly a light-homologation tube while simultaneously destroying any pattern or image being carried in the light beam. Fig. 4, 5 and 6 show different embodiments of the illumination projector 30, particularly considering the arrangement of the light source 32. Those may be combined freely with the different embodiments of an illumination receiving system 39 shown as matters of example.

After the light beam interacted with the yarn 34 moving or being positioned in a measurement gap 9 (see e.g., Fig. 1 or Fig. 2 for structural examples thereof), a shadow might be projected off from the yarn 34 into a direction of the receiving side. Additionally or alternatively, a light might be reflected off from the yarn 34 in other embodiments. The light propagation direction may be referenced as z-direction.

The illumination receiving system 39 can be configured for receiving a shadow projection from a yarn 34 in a textile machine or it can be configured for receiving light reflected from the yarn 34. The illumination receiving system 39 particularly comprises at least one receiving cavity 36 and at least one photosensitive electronical component 37 to form a detector. The receiving cavity 36 can be configured and placed relative to a yarn 34 such that light emitted from a light source 32 and projecting a shadow from the yarn 34 and/or being reflected from the yarn 34 to enter the receiving cavity 36. The light entering the receiving cavity 36 can be transmitted to the at least one photosensitive electronical component 37 as a detector for measuring the incoming illumination. The photosensitive electronical component 37 may also be referenced as a detector. It may be capable of transferring the incoming radiation into an electronical signal which might be analyzed to determine at least one yarn 34 characteristic, such as composition, impurification, defects from yarn 34 formation, etc.

An aperture 35 can be placed at the entrance of the receiving cavity 36. The aperture 35 may limit the light entering the receiving cavity 36, particularly in form of a lighthouse 59 to the light projecting the shadow or light being reflected from the yarn 34. Stray light or light from external light sources not included in the optical setup as described elsewhere herein, may be blocked by the aperture 35. At least the width of an aperture opening 42 of aperture 35 can be set to a range of a minimum length of the defect size of the yarn that is to be measured. Alternatively or additionally, at least the height of the aperture opening 42 of aperture 35 can be set to a range of a maximum defect size of the yarn that is to be measured.

Opposite walls of the receiving cavity 36 may be at least diffusive, non-specular, shaped such that they direct light away from the aperture or are light absorbing in the wavelength range in which the at least one photosensitive electronical component 37 - the photosensitive detector - is sensitive. An aperture of the receiving cavity 36 - not to be mixed up with aperture 35 in the beam path in front of the receiving cavity 36 - may be light absorbing in the wavelength range in which the at least one photosensitive electronical component 37 is sensitive. The surface may be non-specular or shaped or positioned such that it directs light in a direction where potential secondary reflections back into the aperture of the receiving cavity 36 are minimal. This may prevent stray light to reach the photosensitive electronical component 37.

At least one inner surface of the receiving cavity 36 comprises at least one of a non-specular surface finish or a highly reflective surface finish. This may improve the light propagation in the receiving cavity 36.

The exemplary embodiment of a combination comprising an illumination projector 30 and an illumination receiving setup 39, short the receiver, is shown in Fig. 4 and will be described here in greater detail. Therein, both the light source 32, here in form of an LED and photosensitive electronical components 37 can be mounted in plane of the signal processing PCBA 93 (here not shown). This allows that all electrical components can be assembled during a single pass soldering process. The receiving photosensitive electronical component 37, e.g., a photodiode or other type of photosensitive detector, has no direct line of sight to the emitting LED. By this morphology, in which all the electronical components are assembled in plane to the PCBA 93, and where the optical components (illumination projector 30 with waveguide 33 and mirror 21 in mirror housing 31 - see Fig. 3 - and receiving cavity 36 in form of a lighthouse 59) do not require direct assembly to this PCBA 93 but can be attached later during the product assembly process, particularly after the PCBA 93 is placed in the housing body 3. The decoupled assembly of the PCBA 93 and the (passive) optical parts allows a reversed assembly stack up, which shortens the time required for assembly. In addition, it allows undercuts in the design of the housing parts, particularly the housing body 3, which benefits the light projection. These undercuts may form pockets 78 as described with respect to Fig. 11 and shown therein, as well as ribs 27 for stabilizing the housing body 3, but also functioning as spacers for alignment of the respective component to be placed therein. An additional added advantage of the allowance of undercuts in the housing parts is in the sealing design of the housing. All of which are described elsewhere herein.

The illumination projector 30 may consist of a mere tube as waveguide 33. The following may also apply to waveguides 33 of different tape and structure e.g., glass fibers, photonic crystal fibers, etc. Here, the tube is referenced as a mere example. The tube can be simple in structure and may not require special mechanical positioning tolerances, nor any special mechanical size tolerances. The tube can be manufactured as a simple part, i.e. by injection molding as described elsewhere herein. This allows to put a light source 32, particularly an LED, at one side of the tube and receive a light bundle at the other side of the tube with favorable properties of having particularly both: a low divergent angle, and an even distribution and/or no vignetting.

The tube does not require tight mechanical manufacturing tolerances. In one embodiment it can be produced by extrusion and cut to the required length afterwards. The cross section of the tube can be varied according to the requirements: round, elliptical, rectangular, square etc. A particular shape may be elliptical as described in the following.

With an elliptical cross section, the width of tube can be designed according to the desired width requirements of the light bundle and the height of the tube can be designed according to the desired height requirement of the light bundle. The width of the tube and the height of the tube can be individually and independently shaped according to the measurement requirements: the width of the light bundle is particularly adjusted to the width resolution of the yarn defects to be measured. The minimum length of the yarn defect to be detected may determine the width of the tube, whereas the height of the tube is adjusted to the maximum size of the yarn defect to be measured. For a certain illumination intensity, the illumination flux of the light source 32, here the LED, increases according to the cross-section area of the tube, also to be referenced as projection tube. For an asymmetrical measurement requirement where the width of the defect resolution can be less as the maximum defect height (magnitude) a projection tube with an elliptic shape may result in a higher illumination intensity, given the same emitted illumination flux of the light source 32, here the LED, compared to a round tube.

Another advantage of the waveguide 33 as tube to form a projection tube is that the given design can avoid strict tolerances of the emission angle of the light source 32, particularly the LED, other than having a continuous first derivative of its projection profile. It does not even require to have the same angular emission profile in both axis, just a continuous first derivative in either axis. In case of asymmetrical emission axes of the light source 32, particularly as LED, these are oriented in accordance of the dimensions of the waveguide 33 as an illumination projector's 30 tube. The reduced requirements on the emission angles of the light source 32, particularly as LED, allow for a much wider selection range. The wider selection range allows for additional cost optimization.

To achieve these favorable properties of the light bundle, the structure of the inner surface of the waveguide 33 as tube needs to be diffusive and/or non-specular. The length of the tube can (predominantly) determine the resulting divergence angle, whereas the preparation of the internal surface can determine the vignetting of the resulting light bundle. Next to the geometry of the inner surface of the projection tube as described elsewhere herein, the efficiency of the projection is also determined by the reflective properties of the inner surface of the waveguide 33 as tube.

This optical setup can avoid an out of plane arrangement of the electro-optical components (light source 32, particularly as LED, photosensitive electronical component 37 as detector, particularly at least one photodiode) with respect to the processing electronics.

For manufacturing the light source 32, particularly the LED, and the photosensitive electronical component 37, particularly the at least one photodiode, they can be mounted on an additional flex-print (for each of them). Alternatively or additionally a rigid-flex PCB or through-hole components may be used. All these embodiments carry additional costs and complexity during manufacturing. The embodiments described with respect to Fig. 4 can simplify part design and can improve tolerances in their production. Furthermore, assembly and part maintenance as well as service are improved.

The illumination projector 30, producing a light bundle with even distribution (no vignetting) in combination with a low divergent angle, may not be limited to the application of providing a yarn sensor for detecting at least one characteristic of a yarn 34, but can be used in any optical sensor for through light and/or reflective measurements.

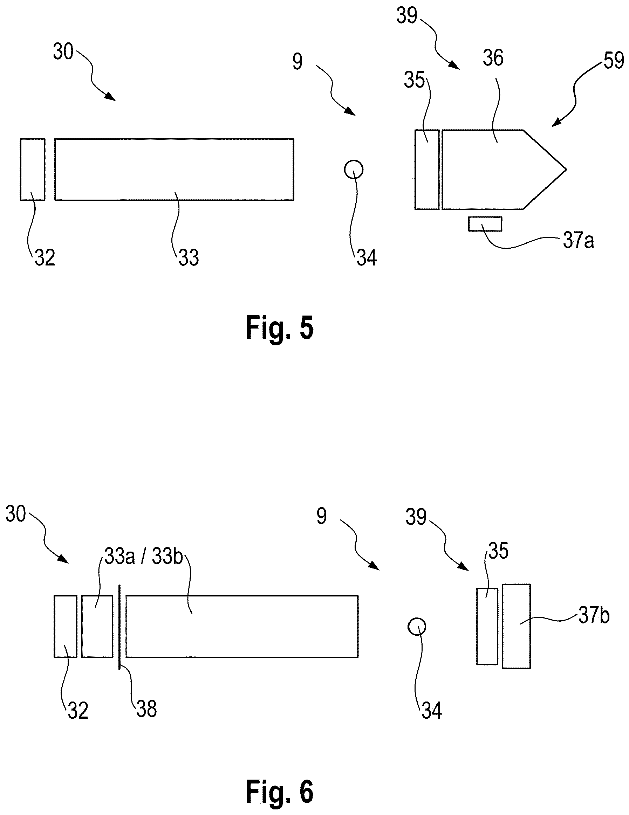

Fig. 5 shows a schematic diagram of an embodiment of an optical setup. In this embodiment an alternative combination of an illumination projector 30 and of an illumination receiving system 39 on the receiving side is shown. Therein, the waveguide 33 is a mere tube as projection tube as well, but with direct attachment of the light source 32, here also an LED, by using a through hole LED component, or where an SMD LED is mounted to a flex print whereby the flex print is attached separately and independently to the signal processing PCBA 93 or where an SMD LED is mounted to the flex part of a rigid-flex board. In each embodiment an additional and individual mounting process of the light emitting part of the light source 32 to the waveguide 33 as a tube may be required. The receiving photo sensitive electronical components 37 have no direct line of sight to the light source 32 as an emitting LED. All the properties described with respect to Fig. 4 for the waveguide 33 as a tube, but also for other types of waveguides 33, remain, except for the 45° mirror 21 (see Fig. 3) in mirror housing 31. The light source 32, here the LED, is no longer mounted in plane of the signal processing PCBA 93. At least the light source 32, here the LED, and the waveguide 33 in form of a tube need to be pre-assembled before continuing with the assembly of the other housing parts.

With respect to both, Figs. 4 and 5, it may particularly be possible to reduce the number of components for the illumination receiving system 39, being beneficial when the photosensitive electrical component 37 has no direct line of sight to the illumination projector 30 as illumination projection system. Without direct line of sight of the photosensitive electrical components 37 to the light emitting components, a diffusor 38 may not be required, as it is placed in the embodiment shown with respect to Fig. 6. Furthermore, to reduce the assembly overhead, the photosensitive electrical component 37 can be placed in plane to the signal processing PCBA 93. Placing all the receiving photosensitive electrical components 37 on the same PCB (to form the PCBA 93) as the signal processing components, the electronics can be assembled in a cost conserving single manufacturing method step, as described elsewhere herein.

The proposed exemplary solution can do both by using a lighthouse 59 as receiving cavity 36. The lighthouse 59 is a receiving cavity 36 in which all the light transmitted into it can be integrated by multiple reflections. The photosensitive electronical component 37 can be mounted to the side of the lighthouse 59 at a position where there is no direct line of sight to the incoming illumination entrapped by the lighthouse 59. This allows the photosensitive electronical component 37 to be placed in plane of the signal processing PCBA 93 without having a direct line of sight to the incoming illumination. Moreover, the lighthouse 59 does not need to be pre-assembled to the single PCBA 93, but this can be done at a later stage in the assembly process as described elsewhere herein. This allows the lighthouse 59 to be placed into the housing first by which an undercut assembly position as pocket 78 of the lighthouse 59 in the housing body 3 is possible. This allows for an optimized optical design with short optical path length at the illumination receiving system 39 and simultaneously allows a housing design to be optimized for ease of assembly.

Placed at such a mounting position e.g., in form of a pocket 78 (see Fig. 11), inside the lighthouse 59 without direct line of sight of the incoming light bundle, the photosensitive electronical component 37 may measure the brightness of the entrapped incoming illumination by the lighthouse 59. Due to this indirect measurement, the size of the photosensitive electronical component can be decoupled and can thus be independent of the size of the object to be measured. This allows for selection of the most suitable photosensitive electronical component 37, with criteria being one or a combination of response time, sensitivity, wavelength range, sensitivity over wavelength, Noise Equivalent Power, active size, packaging type, cost, etc. This selection criteria may be much wider and allows for an improved optimization, where the first criteria to be met can be the maximum defect size that is to be measured.

Fig. 6 shows a schematic diagram of an embodiment of an optical setup. In this exemplary embodiment a waveguide 33 as tube with direct attachment of the light source 32, as LED, by using a through hole LED component, or where an SMD LED is mounted to a flex print whereby the flex print is attached separately and independently to the signal processing PCBA 93 or where an SMD LED is mounted to the flex part of a rigid-flex board, is shown. The light source 32 as LED is no longer mounted in plane of the signal processing PCBA 93. In each of those embodiments an additional and individual mounting process of the light emitting part of the light source 32 to the waveguide 33 as tube is particularly required. At least the light source 32 as LED and the waveguide 33 as tube are particularly pre-assembled before continuing with the assembly of the other housing parts.

The receiving photosensitive electronic component 37 can have a direct line of sight to the light source 32, particularly as emitting LED. The direct line of sight of the photosensitive electronical component 37 to the light source 32, particularly as LED, may require an additional diffusor 38 at the side of the light source 32. Without diffusor 38, the photosensitive electronical component 37 may look straight into the light source, here the LED, and be "blinded" by it. The contours of the light source 32 may be distinguished by the photosensitive electronical component 37 and may affect a uniform sensitivity of yarn defects over the measurement range over the aperture 35. This may be in analogy to a human looking straight into the sun or lamp. For maximizing the working length of the waveguide 33, particularly as a tube, the diffusor 38 may be put immediately after the light source, particularly the LED, in z-direction when propagating from the light source 32. An alternative position can be at the side of the photosensitive electronical component 37, so in the illumination receiving system 39. However, as the photosensitive electronical component 37 may be placed at the exit of the receiving aperture 35, results in inefficient use of the diffusor 38 with the risk of the contour of the light source 32, particularly the LED, may still be noticeable in the measurement field of the photosensitive electronical component 37. Putting the diffusor 38 immediately in front of the photosensitive electronical component 37 before the light bundle hits the photosensitive electronical component 37 may result in the contours of the light source 32, particularly the LED, to be still noticeable by the photosensitive electronical component 37. In the embodiment shown in Fig. 6 a tall detector 37b is used in accordance to the height of aperture 35. In particular, the length of the tall detector 37b may exceed the dimensions of the light beam hitting the aperture 35. In the embodiment shown in Fig. 5 (and respectively in Fig. 4) a small detector 37a may be used which reduces the costs, but also may function at least partially as an aperture, particularly in addition to the aperture 35.

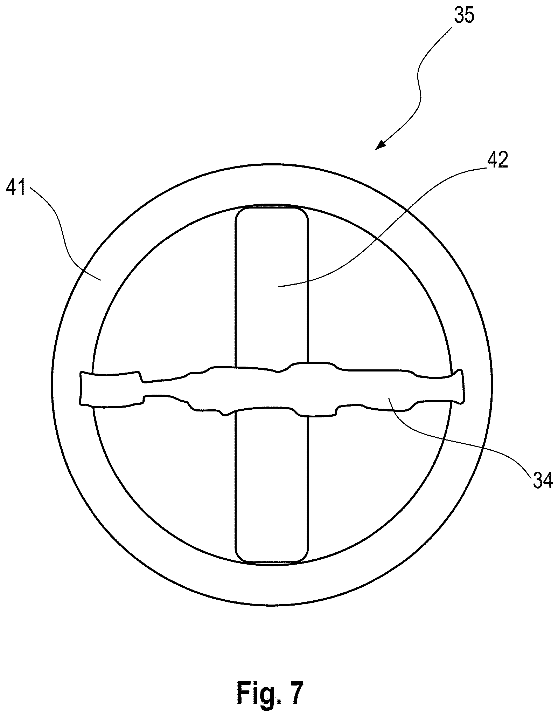

Fig. 7 shows a schematic representation of an embodiment of an aperture 35 with passing yarn 34. The aperture 35 comprises a mounting ring 41 and an aperture opening 42 where the light passes through in z-direction from the light source 32 (here not shown, but see Figs. 4 to 6 for exemplary embodiments). Here, the perspective is in z-direction, thus the light source 32 is behind the viewer. To reduce the sensitivity to environmental illumination conditions, an aperture 35 can be placed at the entrance of the incoming light of the lighthouse 59, as shown in Figs. 4 to 6. To particularly block out unwanted environmental light, the width of the receiving aperture opening can be similar in range as the minimum length of the defect to be measured. The height of the aperture 35 can be in range of the maximum defect size that is to be measured. A thin aperture will allow light from all angles of the opening hemisphere to go through the aperture opening 42.

A thin aperture has no angular blocking. In embodiments where there is a direct attachment of the lighthouse 59 to the aperture 35, a thin aperture is particularly not used as e.g., in the embodiments shown in Figs. 4 and 5. Instead, for effective suppression of environmental light, the aperture 35 is such that it can reduce the opening angle over which light is to enter into the lighthouse 59. The receiving aperture 35 particularly reduces the opening angle of the lighthouse 59 over which light can be received. The exact calculation of the aperture opening dimensions of the lighthouse 59 can take the opening angle of the aperture 35 and the nominal distance of the yarn 34 to the aperture 35 into account.

In Figs. 4 and 5 a thick aperture is used as aperture 35 as a simple-to-implement, low-cost solution. As a numerical example of the opening angle of a thick aperture, a mere mechanical aperture width of 1 mm and a depth of 4 mm- corresponding to the thickness - can have an opening angle of 28°. Any light bouncing off from the opposite wall of the illumination receiving system 39 in the direction of the lighthouse aperture 86 within the 28° reception conus can pass through the aperture 35. Regardless of the exact dimensions of the opening of a thick aperture, it is therefore beneficial to design the opposite walls of the illumination receiving system 39 to be non-specular, particularly with a shape that directs light away from the receiving aperture and which may be light absorbing in the wavelength range in which the photosensitive electronical component 37 is measuring. Therefore, it may be coated with a carbon component to render it black. Furthermore, to avoid secondary reflections, the aperture of the receiving cavity 36 itself can be light absorbing in the wavelength range in which the photosensitive electronical component 37 is measuring. The aperture of the receiving cavity 36 may be non-specular and may direct light in a direction where potential secondary reflections back into the aperture of the receiving cavity 36 are minimal. When using a lighthouse 59, the embodiments can comprise a thick aperture in combination with anti-secondary reflective shapes of the lighthouse aperture 86 and of other opposing structures, particularly combined with a light-absorbing coating or respective light-absorbing materials of these structures.

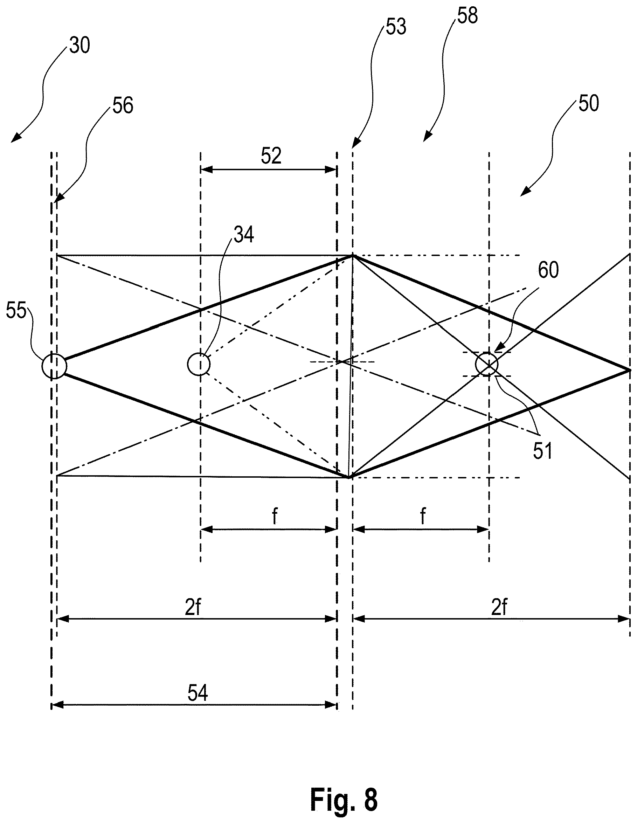

Fig. 8 shows a schematic ray diagram of an embodiment of an optical setup. Therein, a thin aperture 53 with lens assembly can be placed in front of the entrance of the receiving cavity 36 in an f-configuration for a shadow projection. The aperture opening 42 of aperture 35 is schematically represented as aperture diameter 51. Even though the aperture diameter 51 is referenced, the respective aperture opening 42 does not have to be round as described in detail elsewhere herein.

In case of a shadow projection, alternative embodiments to those described with respect to Figs. 4, 5 and 7 may combine a lens with a single aperture to further enhance the Signal to Noise ratio (S/N). A thin aperture with the desired sizes according to the defect to be measured can be in close spatial proximity to a focusing lens. The thin aperture together with the lens may form a thin aperture with lens assembly 53 which may be placed in a wall of the measurement gap 9, opposite to the illumination projector 30. The focal length of the lens can be selected such, that the focal length f is half the measurement gap width 54, the width of the measurement gap 9. The collimated light from the illumination projector 30 impinges onto the aperture 35 with lens assembly 53 and is focused by the lens on its focal point being placed inside the opening diameter 51 (the thin straight lines in Fig. 8). Stray light (dash-double-dotted lines in Fig. 8) particularly originates from the yarn 34 may impinge onto the aperture and may be converted to a parallel (collimated) beam of light by the lens. Stray light originating from the outer bounds of the illumination projector objects 56 (dash-dotted lines in Fig. 8) can pass the aperture with lens assembly 53 particularly having the same angle as that with which they were impinging. Stray light particularly originating from the center 55 of the illumination projector objects 56 (thick straight lines in Fig. 8) may pass the aperture and may be focused by the lens at a distance of 2f behind the lens assembly 53. From the ray traces in the Fig. 8, providing a simple pin-hole with aperture diameter 51 at the entrance to the lighthouse 59 at the position of the secondary focus point 60 of the lens assembly 53 can remove most of the stray light projections originating from the opposite wall (dash-dotted and straight lines in Fig. 8) as well as from the yarn 34 (dash-double-dotted lines in Fig. 8). The part holding the thin aperture with lens assembly 53 may be connected by a tube to the lighthouse 59, the interconnecting tube 58.

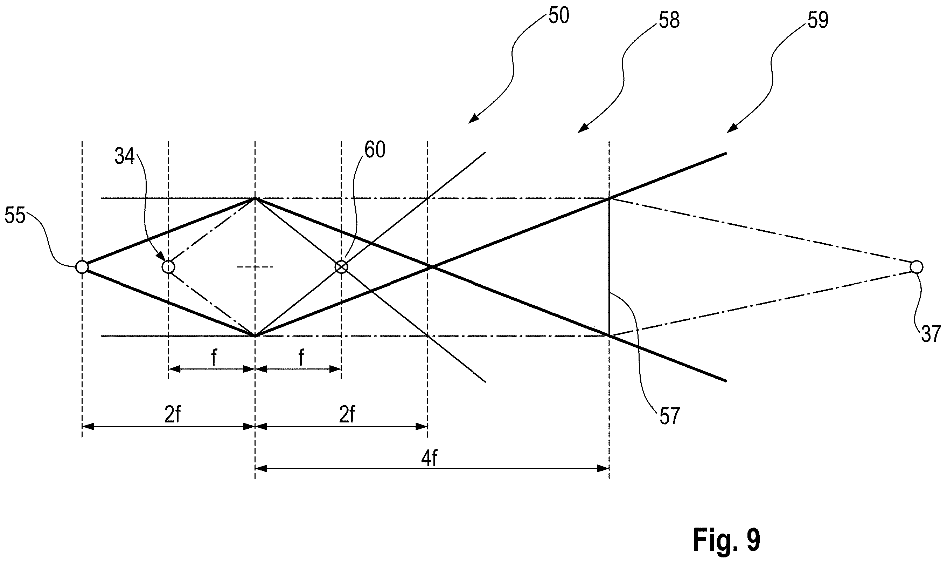

Fig. 9 shows a schematic ray diagram of an embodiment of an optical setup. Therein, a thin aperture with lens assembly 53 can be placed in front of the entrance to the receiving cavity 36 in a greater than 2f configuration, particularly mounted in an interconnecting tube 58, for a reflection arrangement, particularly wherein the aperture opening size of the aperture of the receiving cavity 36 matches the aperture opening 42 size of the thin aperture of the thin aperture with lens assembly 53.

In case of a reflective arrangement, the embodiment shown in Fig. 9 and described in the following increases Signal to Noise ratio (S/N). The reflective arrangement can use the same thin aperture with lens assembly 53 as was described with respect to the shadow projection arrangement from the embodiment depicted in Fig. 8, but the entrance to the lighthouse 59 may be positioned at a distance further then 2f from the lens and the entrance 57 to the lighthouse 59 may have the same opening as the thin aperture, particularly the aperture diameter 51. The distance between the lighthouse 59 and the lens may be at least 4f.

Light reflected off the yarn 34 (dash-dotted lines) after passing through the aperture 35 with lens assembly 53, can be converted by the lens to a beam of parallel light rays (collimated beam). This light beam can pass the entrance to the lighthouse 59. Parallel (collimated light) back reflected from the opposite wall (thin straight lines in Fig. 9) are, after passing the aperture of the aperture with lens assembly 53, focused to the secondary focus point 60 of the lens. Because of their steep angle, these rays can be absorbed by the walls of the interconnecting tube 58 between the lens and the entrance 57 of the lighthouse 59. Stray light, seemingly coming from the center 55 of the opposite wall (straight thick lines in Fig. 9), are, particularly after passing through the thin aperture, focused at a distance 2f from the lens by the lens of assembly 53. Because of the focus angle, this light spot will increase to the size of the thin aperture at distance 4f from the lens.

In other words, the embodiments described can provide the means for implementing a method for projecting a shadow off from a yarn or for reflecting light off a yarn 34. The method may comprise the steps of providing light emitted by a light source 32. At least a part of the light emitted by the light source 32 may be collected in a waveguide in a method step. The light from the waveguide 33 may be transmitted to a yarn.

Alternatively or additionally, the embodiments described can provide the means for implementing a method for measuring at least one characteristic in a yarn e.g., yarn defects. The method can comprise the step of emitting light from a light source 32 to a yarn 34 such that the light projects a shadow from the yarn 34 and/or the light reflects from the yarn 34. In a step the light with the projected shadow or reflected from the yarn 34 can be collected in a receiving cavity 36. In a step the light entering the receiving cavity 36 can be transmitted to at least one photosensitive electronical component 37 such that the incoming illumination is to be measured.

Fig. 10A shows a top perspective view of an embodiment of a dual use light guide 70 with uses of signaling to the operator and the ability to provide operator feedback by means of a button press action. Light guide 70 consists of an operator activatable head 8 connected to a light guide shaft 71 for conducting light from a signaling light source, particularly a signaling LED (not shown).

The light guide shaft 71 and head 8 are particularly placed on a basis 72, wherein the light guide shaft 71 particularly forms a shaft to transmit the force of pushing the head 8. In a particular arrangement, head 8 is used for distribution of the light transmitted by light shaft 71 in a preferred direction to the operator, and in which basis 72 is used to efficiently collect light from the signaling LED (not shown). In particular, light guide 70 consisting of head 8, light shaft 71 and basis 72 and in which head 8, light guide shaft 71 and basis 72 are optically interconnected and constitute a single part.

For indicating the state of the clearer 10 to an operator, indicating lights can be used. These indicating lights can be implemented by one or multiple LED's. The LED's can have single color or consist of multiple colors. A typical intuitive color scheme when running may be green for OK and red for FAULT (corresponding to traffic light colors). Besides these states there can be many other states of which the operator is to be informed: length of yarn to be removed, clearer blocked, incorrect yarn parameters, communication faulty etc.

An indication method may be implemented based on using blinking schemes of the LEDs used for indication. The blinking period and blinking scheme can indicate the state. The blinking scheme can extend to all the LED's available for indication use, conveying morse-type signs to the operator. Even though the messaging is less-intuitive as display communication and may take some initial training to get familiar with the morse-type code, its implementation is beneficial because of the variance of timing schemes. Moreover, LEDs are visible and distinguishable from a far distance. Furthermore, displays are more costly and more demanding in space, maintenance and implementation.

Instead of a display, using multiple LEDs or a couple of LEDs combined with a blinking scheme, particularly a signaling RGB LED is proposed by which the most important states can be easily indicated by intuitive color coding. With the RGB LED traffic light signaling as well as other states can be implemented. With the inclusion of a single timer on the signaling RGB LED the number of states can be doubled, moreover closely related states or problems can be indicated by "color + non blinking" and "color + blinking". For instance, three different colors to indicate the amount of yarn 34 that is to be removed (short, middle, long) can be used. An alternative can be to choose a color in combination with a timing sequence to indicate the amount of yarn 34 that is to be removed. In these signaling schemes, either a positive or a negative regime can be adhered to. A positive scheme may indicate the amount of yarn that is to be removed, a negative scheme may indicate that yarn is to be removed until all the yarn that is to be removed has been removed. In a positive scheme, "short blinking" may correspond to "short fault", "little yarn to be removed", "middle period blinking" may correspond to "middle fault", "several meters of yarn to be removed", "long period blinking" may correspond to "long fault", "considerable yarn needs to be removed (i.e. 10m or more)". In another embodiment adhering to a negative regime, the signaling LED may keep indicating yarn to be removed for as long as there is still yarn to be removed.

The signaling LED can be linked to a light guide 70. The shape of the light guide 70 may be such that it emits an elliptical illumination pattern: the emission flux side wards to the adjacent spindles are considerably larger compared to the emission angles in up (ceiling) and down (floor) direction. This emission pattern gives better visibility to the operator from far and reduces power consumption or necessity of LED loading to do so. Both improve efficiency. Further to the shape of the dome at the top of the light guide 70, ellipsoidal Fresnel lenses or other structures impressed to the light emitting surface of button 8 (particularly its head) can be incorporated in addition to improve light distribution into the desired direction. Another feature for improvement of the light guide's 70 efficiency is to adapt the surface of the light guide 70 at the basis 72 where the light of the LED is coupled into, according to the emission profile of the signaling RGB LED and the desired light distribution i.e., typically domed as well, alternatively a conus shape. As shown in Fig. 10C, wherein a perspective bottom view of an embodiment of a light guide 70 is depicted, stopper 73 may be in place. When using light guide 70 as a button 8, stoppers 73 constraint the movement of the head in downwards direction to the side wall of the PCBA (not shown) and prevent over exerting pressure to a (micro) switch that is assembled to the same PCBA. Incorporating stoppers at both sides of light guide 70, reliably prevent overexerting the (micro) switch also in case of tilted activation of head 8. The basis 72 may also engage with an LED, particularly in-between the stoppers 73, to couple light into the light guide shaft 71.

Besides indicating the state of the clearer 10 to the operator, often the clearer 10 (or its control system, control function and/or computer program products) desire to receive feedback from the operator, for instance in form of an acknowledgement or a confirmation that a certain corrective process or handling has been completed or can be initiated. To get the feedback from the operator a button 8 can be integrated into the clearer 10. The button 8 is particularly an additional component that can be placed onto the housing, particularly the housing body 3. In the housing provisions can be made for the button 8 to be holstered. Furthermore, the parts can be produced, assembled and checked.

Integrating the button function together with the indicating light guide 70 can help reducing costs. A simple and robust alternative exemplary embodiment is that of a mechanical solution requiring the light guide 70 to be movable. The light guide 70 may be pushed down by the operator if so required and retracts on its own upon release. A mechanical solution is particularly independent of the means by which the button 8 is pressed. A mechanical solution is able to give tactile feedback to the operator pressing it.

An alternative exemplary embodiment to a mechanical button 8 can be an all-optical solution. In an all-optical solution, an additional photodiode may be used with which the variance in brightness of the returned or back-reflected light of the light guide 70 upon activation and/or blocking and/or reflection by the operators hand of the light guide 70 is measured. In this embodiment moving parts are avoided, which reduces mechanical maintenance requirements. Therefore, no tactile feedback can be given and the function may be dependent on environmental circumstances (sunlight exposure).

Fig. 10B shows a top perspective view of an exemplary embodiment of a button 8 with a seal dome 7. For embodiments implementing the mechanical button 8, the light guide 70 can be capable to move down when being pressed by the operator and can be capable to retract on its own upon release. The spring-loaded action for the light guide 70 to retract on its own can be implemented by the sealing. The sealing may comprise a seal dome 7, which compresses when being pressed which results in storing energy in a material deformation used to return to its original shape when released. The tactile feedback can be achieved by activating a micro-switch at the end of the stroke of the light guide 70. To ensure the micro-switch is not over-pressed by the operator, a mechanical stopper 73 may be integrated into the PCBA 93 (here not shown) to which the tactile switch is assembled. By implementation of the stopper 73 on the same PCBA 93 as the tactile switch, all tolerances can be controlled during PCBA 93 manufacturing particularly reducing cost and increasing accuracy. Introduction of tolerances during assembly or other tolerance stack ups can thus be avoided.

A basis 72 can be integrated as a flange underneath the button and/or the light guide that is significantly wider compared to the opening in the housing body 3 where the button 8 or the button with seal dome 7 is to be implemented. The light guide 70 and/or button 8 can be assembled together with its seal dome 7 from the inside of the housing prior to assembly of the PCBA 93.

Through the integrated flange, the button 8 to the outside is constrained by the housing body 3 and to the inside by the PCBA 93 (after its assembly). This can reduce the risk that the described button 8 (alone or together with a seal dome 7) can inadvertently be removed from the housing body 3 e.g., by a fingernail gripping in the seam (not shown) between housing body 3 and button 8 or between seal dome 7 and housing body 3.

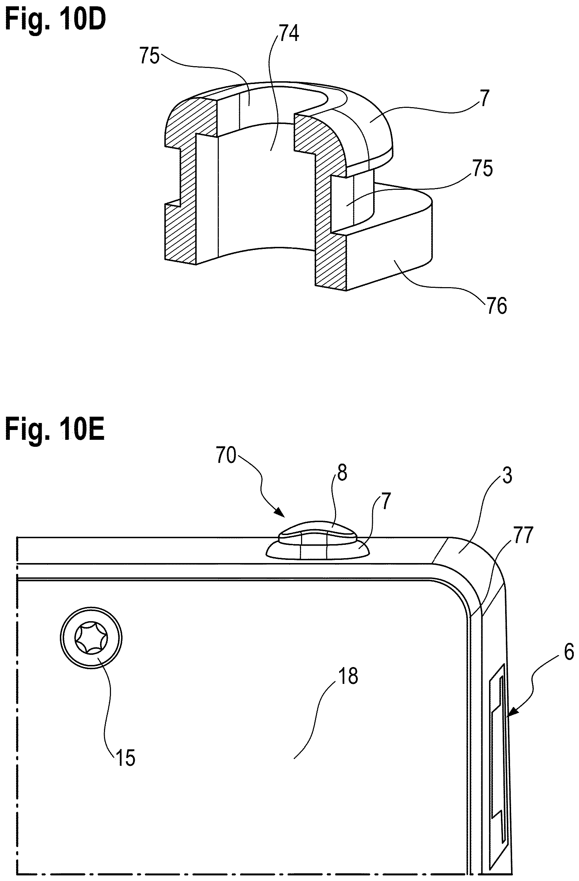

Fig. 10D shows a partial cut view of an exemplary embodiment of a seal dome 7 for a button 8 as described with respect to Figs. 10A to 10C. The seal particularly comprises a hollow core 74 that can be configured and positioned for the light guide 70 to be incorporated therein. The button 8 can be surrounded in a circumferential direction by the seal dome 7. A spring part 75 can support the spring action by deforming upon pressing the button 8, storing energy in the material deformation to return to the original unpressed configuration upon release of the pressure on the button 8. Furthermore, a seal basis 76 can be provided to insulate the button 8 and/or the light guide 70 mechanically and electronically from the PCBA 93 (not shown).

Fig. 10E shows a button 8 with seal dome 7 surrounding it, placed as button with light guide 70 at a housing body 3. The perspective view is onto the bottom of the housing, where the housing cover 18 is sunk into the housing body 3 as described elsewhere herein. A screw is shown being located in a screw hole 15 to compress the housing cover 18 into the housing body 3, forming a seam 77. Thus, the button may be positioned such that it protrudes on a sidewall of the housing body 3 when placed in textile machine.

Fig. 11 shows a cut view through a plane parallel to the yarn propagation direction and parallel to a z-direction through a housing. The window carrier 17 can be held by compression between the housing body 3 and the housing cover 18 forming a linkage between the housing body 3 and the housing cover 18. The linkage particularly comprises no sealing compound or seal which seals the parts contributing to the housing. The linkage particularly comprises no glue which glues the parts contributing to the housing. Furthermore, no electronics are connected to the housing.

To prevent dust ingress into the housing the clearer 10 can meet dust protection rating IP5X, particularly IP6X. Therefore, the embodiment depicted in Fig. 11 comprises a sealing that is a combination between form consistent part design and a meandering sealing.

According to the invention, the housing cover 18 comprises a gravity seal. To do so, the clearer 10, the housing body 3 and the housing cover 18 are configured such that the housing cover 18 may be arranged at the bottom of the clearer 10, latest when implemented into a textile machine for operation. In addition, the housing cover 18 is particularly sunk into the housing body 3. Therefore, there are particularly no cover seams 77 to the side of the housing body 3 only underneath as shown in Fig. 10E for matter of example. By close form fit of the housing cover 18 to the sidewalls of the housing body 3, a first seal can be created. By placing the housing cover 18 at the bottom and sunken into the housing body 3, all dust falling in direction of gravity, cannot enter into the housing, because of gravity (gravity seal).

Further for avoiding of dust to penetrate though the housing cover's 18 seal, a double meandering sealing 79 with form fit can be employed. To penetrate the housing cover's 18 seal, dust would have to move up (against gravity) between the housing body's 3 side wall 69 and housing cover 18, particularly up to a first recess of the housing cover 18. There, the dust would have to move horizontally between housing body 3 and housing cover 18 until reach a first wall 90. There the dust would have to move up again (against gravity) between housing body 3 and housing cover 18, followed by a horizontal movement at a next recess in the housing body 3 (the top of the first wall 90), then down in direction of gravity into a valley 91 before reaching a second wall 92 when migrating in horizontal direction, where it would have to move up one more time again against gravity. Because of the tight form fit and the compression of the housing cover 18 to the housing body 3, particularly by screws, this sealing may achieve an IP6X dust protection rating.

The same sealing technique can be employed for sealing the window carrier 17 to the housing body 3 at the top as well as sealing to the housing cover 18 at the bottom. For sealing of the window carrier 17 to the front or to the stern side of the clearer an even more elaborate meandering seal is implemented: by using two orthogonal sealing features as shown in Fig. 12B.

Therein, the dust would have to move first sideways to the back of the clearer 10 and/or of the housing body 3, then horizontally to the side of the housing body 3, followed by a forwards movement, followed by again a horizontal movement, then followed by a repeated movement to the back of the housing after which another horizontal stretch needs to be passed followed by a third movement to the back succeeded by fourth horizontal movement and a fourth movement to the back after which a reversed sideways stretch is to be passed and then a final fifth movement to the back. This is also shown in the Fig. 2, where the window carrier 17 is shown from a top angled view. In this and alternative embodiments several angles as well as reversed motion directions are provided. These angles and reversed motions do not have to be full U-turns as described above. Alternative, several different angles can be chosen and also several meandering length of the path provided for the dust to pass. The particular scheme may be provided such that it achieves the desired dust protection rating. The embodiment of the double meandering seal 79 as shown in Figs. 11, 12A and 12B, particularly in concatenation with Fig. 2, can achieve IP6x dust protection rating as it is defined on the date of filing or the date of priority. Fig. 12A shows a cut view through a first plane perpendicular to the yarn propagation direction through a housing showing a selected part thereof. Fig. 12B shows a cut view through a second plane perpendicular to the yarn propagation direction through a housing showing a selected part thereof. In both, Fig. 12A and Fig. 12B meandering sealings are shown that block dust from entering the housing body 3 due to a form fit between the housing body 3 and Window Carrier 17 and housing cover 18.

Fig. 12A refers to the double meandering sealing 79 as described with respect to Fig. 11, just in a bottom view, not in a cut view. The gravity seal is formed by the housing cover 18 (here not shown) sunken into the housing body 3 to be oriented such that the housing cover 18 forms the bottom of the housing. In this and alternative embodiments several angles as well as reversed motion directions are provided. These angles and reversed motions do not have to be full up-down-turns as described above. Alternatively, several different angles can be chosen and also several meandering length of the path provided for the dust to pass. The particular scheme may be provided such that it achieves the desired dust protection rating.

Fig. 12B shows the stern side sealing arrangement with orthogonal locking and sealing arrangement 83 that forms a meandering seal as described already with respect to Fig. 11 for the window carrier 17. In this and alternative embodiments several angles as well as reversed motion directions are provided. These angles and reversed motions do not have to be full U-turns as described above. Alternative, several different angles can be chosen and also several meandering length of the path provided for the dust to pass. The particular scheme may be provided such that it achieves the desired dust protection rating.

The above-described embodiments prevent the use of individual and separate sealing components which would have to be placed between housing parts such as the housing body 3 and the housing cover 18. The use of individual and separate sealing components between housing parts may require their production and transport to the assembly position. In case of an elastomer, the elastomer would have to be selected, produced, transported and subsequently assembled. The fitting of an elastomer often comes with issues of form fit: A bit to short and the sealing tends to jump out because of the stretching and tensioning, a bit too long and there is a tendency to buckle following from the surplus material's tendency to form a buckle which is potentially cut when tightening the housing parts, potentially weakening or destroying the sealing component.

During field service the individual and independent elastomer seal would undergo aging of the material. Thus, the material might lose elasticity when compared to the elasticity it had during production. It potentially also provides sticking issues to a housing part, etc. Also, upon opening of the housing, the individual and independent seal, although visibly in shape, might have lost at least some level of its former elasticity which causes a risk after re-tightening of the housing.

When using an independent and individual sealing compound production is costly. During servicing, the independent and individual sealing compound may need to be broken or opened. This can be done by heating for example. During heating, it would have to be ensured, that the storage temperature range of the clearer 10 is not exceeded, to reduce the risk of degradation of the housing material and or of internal component alignments. All these drawbacks may be circumvented by the embodiment as described above.

Fig. 13A shows a cut view through a first plane perpendicular to the yarn propagation direction through a housing shown in full, partially assembled. Fig. 13B shows an embodiment of a window carrier 17 with an embodiment of a lighthouse 59. Therein, eyelets 2 can guide and stabilize the yarn 34 in a measurement gap 9 of a clearer 10. The eyelets 2 may be placed in the window carrier 17. Alternatively, the eyelets 2 can be placed at the housing body 3 for one part and at the housing cover 18 as the other part. Fig. 13C shows an embodiment of a lighthouse 59. Fig. 13D shows a cut view through a first plane perpendicular to the yarn propagation direction through a housing shown in full, partially assembled with an optical setup placed therein.

Figs. 13A to 13D show a partial representation of a method of assembling a housing in their respective order. The point of view is from a bottom into the housing body 3. The housing cover 18 is not sunk into the bottom of the respective housing body 3, yet. Therein, the step of placing optical components in a housing body as shown in Fig. 13A and concluded in Fig. 13D is shown.

Prior to assembling the optical components to set up an optical setup, the button 8 as button with light guide 70 may be placed into a fitting hole in the housing body 3 and sealed using the seal dome 7 with seal basis 76. The placing of the optical components can be followed by placing a PCBA 93 in the housing body 3, further followed by a step of connecting the optical components and the PCBA 93.

In doing so, no optical (fine) alignment is to be performed on the optical components to be linked with the PCBA 93 except for mounting the optical components to the same plane on the PCBA 93, particularly giving already the only required course alignment.

The window carrier 17 can be placed in the housing body 3 as shown in Fig. 13A. In case of an optimized illumination path with an undercut of the lighthouse 59 to the window carrier 17 it might be provided during assembly to have a combined insertion process for lighthouse 59 and window carrier 17 (Fig 13 B). The lighthouse 59 is shown in Fig. 13 C in isolation. Therein, a holder 80 may provide a screw hole 15 to screw the lighthouse 59 to the PCBA 93 at a later stage during assembly. An alignment pin 87 provides the necessary alignment, particularly together with the ribs 27 as they were shown in Figs. 12A and 12B for example to align the optical as well as the electronical components. The PCBA 93 may place in the alignment pin 87 and thus align receiving photo sensitive electronical component 37 (here not shown as the PCBA 93 is not in place).

Because the housing particularly has no individual and separate sealing component or seals made out of elastomers, silicons and or rubbers the housing can be assembled or disassembled multiple times and particularly because of using only screws, the assembly or disassembly can be done with ease. Together with the undercut of the housing forming pockets 78 as shown and explained with respect to Fig. 11, no special alignment has to be performed. Furthermore, no aging processes take place. This allows for completion of the complete assembly in one go. As the whole assembly can be done at a single stage, this saves valuable floor space in the production facility. Moreover it can save on production effort: No part is handled twice. This saves further resources. As an additional benefit, the assembly process facilities automation during production.

As described elsewhere herein, all electronics are on at least one PCBA 93, particularly a single PCBA 93. Alignment structures 16 can align the PCBA 93 with the optical components. The alignment tolerances of the waveguide 33 as projector tube and lighthouse 59 with aperture 35 (here not visible) are accommodating. This may avoid the need of a special alignment procedure other than mounting them to the same plane on the PCBA 93 and particularly ensuring general alignment by placement of pinholes in the mounting plane PCBA 93. The alignment by alignment structures 16 on the PCBA 93, but also by the ribs 27 and the formed pockets 78, the optical components such as the waveguide 33 and/or the lighthouse 59 can be placed such that they already align with the PCBA 93 before it is even introduced to the housing body 3. A beam entrance 88 of the waveguide may align with the light source 32 (not shown), while a beam exit 85 of the lighthouse 59 may align with the photosensitive electronical component 37 (not shown in Fig. 13D).

For improved serviceability as well as increased throughput during assembly, particularly only screws are used, but no gluing, as already described elsewhere herein. Because no special alignment requirements are mandatory due to the structural design, the optical components can be placed individually into the housing body 3 first and only connected to the PCBA 93 after the PCBA 93 has been assembled into the housing body 3. This reverses any assembly process where PCBA 93 and optics are assembled to a sub-module first. To the contrary, in the depicted exemplary embodiment, the optics are placed into the housing body first (after the button 8 is placed, but this can be independently performed, unless it is placed before the PCBA 93 is placed). This allows to implement undercuts as pockets 78 (see Fig. 11) in the housing body for placement of the optical components. This in turn allows for independent optimization of the design of the optical components and the sealing design of the housing as was described in detail herein with respect to Figs. 11 to 12B.

In another step (not shown), the housing cover 18 is sunk into the housing body 3, particularly following a step of placing the PCBA 93. There might be no intermediate testing of any electronical sub-modules, as all the required tests can be conducted on the final assembly. Should a problem be detected, disassembly of the housing is quite fast after which the problem can be fixed (rework loop). By this process, assembly throughput is increased, the number of skilled operators as well as the required floor space in assembly may be reduced and production automation may be facilitated.

As a summary, the method for assembly of a housing or a clearer may comprise the following steps: The housing can be placed in an assembly fixture. The sealing 5 for the connector 6 and/or sealing dome 7 for button 8 can be inserted, followed by inserting the light guide 70 and/or button 8. These steps can also be reversed. The window carrier 17 may be put together with the illumination receiving system 39 as an insertion combination into the housing body 3 (sliding and pressing the window carrier 17 into the housing body 3: light compression may exist, even though it is not screwed in yet). During this process normal production cleanliness can be sufficient. Windows 19, 89 of the window carrier 17 can be checked on obvious dirt / smear prior to insertion. It can further be checked that no foreign particles are in the receiver cavity 36 and that the aperture 35 is open, having the intended shape and structure. Sub-assemblies may be provided in that the lighthouse aperture 86 may be pushed into the lighthouse 56 and in that the eyelets 2 may be heat staked to the window carrier 17. The illumination projector 30 as assembly may be put into the housing body 3. The illumination projector 30 as assembly can be checked on cleanliness and that no foreign particles, no scratches and no deformations are present in the waveguide 33, particularly in form of a tube. Photodiode and LED holes can be checked that they are open, not blocked, no flashes etc. are present. Sub-assemblies may be provided e.g., by pushing the projector aperture to the illumination projector's 30 core of the waveguide 33 being a tube and/or by heat staking of the mirror 21 to the illumination projector's 30 core of the waveguide 33 being a tube. The connector seal 5 into the housing body 3 to implement the connector plug 6. In all these steps one may ensure that the corners of the components and sub-assemblies are seated correctly. The PCBA 93 (not shown here) may be slid (from the right or from the bottom) into the housing body 3. In a step it may be taken care of that the seating of the connector seal 5 is placed correctly. In a step the illumination projector 30 (projector) and illumination receiving system 39 (receiver) using may be screwed to the housing body using at least one screw each, particularly by using only a single screw each. In a step one may take care of a correct alignment of each of the pins to the PCBA 93. The PCBA 93 may be secured to the housing body 3, particularly by using at least a single screw, particularly by using only a single screw. The housing cover 18 is sunk into a bottom of the housing body and may be secured, particularly with 3 screws.

A preferred method for manufacturing of the projection tube is particularly by (direct) injection molding, particularly without secondary inner surface conditioning processes, like secondary grinding (machining) or coating of the inner surface of the waveguide 33 being a tube. For secure tool release of the inner pin shaping the inner surface and its structure, a draft angle may be provided. Suitable draft angles can range between 1 and 10 degrees. Depending on the cross section of the inner tube and the desired structure the draft angle can range between 3 and 7 degrees. The inclusion of the draft angle can have a minimal influence on the divergence angle of the exit beam and particularly only a minor can have an influence on the intensity of the exit light bundle.

For direct injection molding, the material choice may affect the efficiency of the embodiment. The material can allow for an inherently non-specular and highly reflective (90 % or higher, preferably 99 % or higher) surface finish. The non-specular surface requirement can be influenced by the surface finish of the molding tool and by the properties of the material used (filler properties). A material that is highly reflective and non-directional reduces the requirements to be given for the emitted illumination flux of the light source 32, particularly the LED. A material providing very high reflective properties and being non directional can be is Barium Sulfate (BaSO₄).

Manufacturing of the lighthouse 59 can be performed by direct injection molding (particularly no secondary processes like machining and or coating of the inner surface of the lighthouse 59). The light entrapped by the lighthouse 59 can be integrated by multiple non-specular reflections as described elsewhere herein. The material choice of the lighthouse 59 can affect the efficiency of the embodiment resulting from the method. The material can have an inherently non-specular and highly reflective surface finish. The non-specular surface requirement is influenced by the surface finish of the molding tool and by the properties of the material used (filler properties). A material that is highly reflective and non-directional increases the illumination efficiency. A material providing very high reflective properties and being non directional can be Barium Sulfate (BaSO₄).

Barium Sulfate can be provided as a powder. When sprayed directly to a surface it can easily be rubbed off. Therefore, Barium Sulfate can be added to a coating (for instance latex with 50% weight/weight also in short w/w), improving its rubbing fastness. Providing barium sulfate in a coating can be achieved by submitting the receiving cavity 36 for secondary processing. Secondary processing of the receiving cavity 36 might add undesirable costs during manufacturing.

A lower cost application alternative is provided in that Barium Sulfate pigments can be added to a base plastic compound and by extruding this mixture. The resulting granulate can be used for injection molding in method steps described elsewhere herein. Precipitated Barium Sulfate may be used as a delivery component. Depending on the other properties the receiving cavity 36 can be provided with, the filler can be mixed with the base material of choice, like i.e., PA or ABS. Moreover, Barium Sulfate as a filler can add further properties to the base material. It may improve processability, hardness, and chemical resistance. As an alternative to Barium Sulfate, Titanium Dioxide (TiO₂) can be used as a highly reflective filler for a plastic compound.

Opposite to the beneficial highly reflective properties of the lighthouse 59, for a thick aperture as well as for the interconnection tube 58 between the holder 80 of the lens with thin aperture (the thin aperture with lens assembly 53) and lighthouse 59, a material can be selected that has highly absorptive properties. A material with a high carbon content can be highly absorptive over a wide wavelength range. Moreover, when used as a filler in a plastic compound, it may render this compound conductive. As such, adding carbon as a filler to the material choice for the aperture may reduce environmental and stray light sensitivity and may also improve EMV and ESD susceptibility.

In particular, the term "may" refers to optional features of the invention. Consequently, there are also further aspects and/or embodiments of the invention which additionally or alternatively have the respective feature or features. Terms like "first", "second", "third" may be used to refer to a list of elements, but does not necessarily describe these features or elements according to their importance, their order of appearance or importance. Therefore, these elements or features may specify the different aspects in any other particular order, unless explicitly expressed.

**List of references**

| | | | |
|---|---|---|---|
| 1 | air venting hole | 31 | mirror housing |
| 2 | eyelet | 32 | light source as LED |
| 3 | housing body | 33 | waveguide as light homologation tube |
| 4 | mounting element | | |
| 5 | connector sealing | 33a | short light homologation tube |
| 6 | connector plug | 33b | long light homologation tube |
| 7 | seal dome | 34 | yarn |
| 8 | button | 35 | aperture |
| 9 | measurement gap | 36 | receiving cavity as integrating tube |
| 10 | yarn clearer | 37 | photosensitive electronical component as detector |
| 11 | yarn guiding device as yarn guide | | |
| 12 | yarn exit guide | 37a | small detector |
| 13 | connector pins | 37b | big detector |
| 14 | contact surface for window carrier on housing body | 38 | diffusor |
| | | 39 | illumination receiving system on receiving side |
| 15 | screw holes | | |
| 16 | alignment structures | | |
| 17 | window carrier | 41 | mounting ring |
| 18 | housing cover | 42 | aperture opening |
| 19 | window on receiving side as second window to be passed by the light | 50 | optical setup |
| | | 51 | aperture diameter |
| 21 | mirror | 52 | focal distance |
| 22 | heat stacks | 53 | thin aperture with lens assembly |
| 23 | engagement pin | 54 | measurement gap width |
| 24 | spacers | 55 | center of illumination projector object |
| 25 | recess | 56 | illumination projector object |
| 26 | tube | 57 | entrance to lighthouse |
| 27 | ribs | 58 | interconnecting tube |
| | | 59 | lighthouse |
| 30 | illumination projector on an emission side | 60 | secondary focus point |
| 69 | side wall | | features |
| 70 | light guide | 85 | beam exit |
| 71 | light guide shaft | 86 | lighthouse aperture |
| 72 | basis | 87 | alignment pin |
| 73 | stopper | 88 | beam entrance |
| 74 | hollow core for light guide | 89 | window on emission side (schematic representation) as first window to be passed by light |
| 75 | spring part | | |
| 76 | seal basis | | |
| 77 | seam | 90 | first wall of double meandering sealing |
| 78 | pocket | | |
| 79 | double meandering sealing | 91 | valley of double meandering sealing |
| 80 | holder | 92 | second wall of double meandering sealing |
| 83 | orthogonal locking and sealing | 93 | PCBA |

## Claims

1. Housing of a clearer (10) for a textile machine configured for guiding a yarn (34) to optically detect characteristics in the yarn (34), the housing comprises:
- at least a first housing part formed by a housing body (3) and a window carrier (17), wherein the window carrier (17) is configured and arranged in a first configuration at the first housing part for a light from a light source (32) to pass at least one of a first window (89) for illuminating a guided yarn (34) or a second window (19) to pass the light from the guided yarn (34) to a receiver;
wherein the window carrier (17) is detachable from the housing, particularly reversibly detachable from the housing, for transition to a second configuration, particularly for cleaning at least one window,
a housing cover (18) as second housing part for covering and/or sealing the housing body (3), the housing being **characterized in that** it further comprises: a gravity seal formed by the housing cover (18) sunken into the housing body (3) to be oriented such that the housing cover (18) forms the bottom of the housing.

2. Housing according to claim 1, **characterized in that** the housing comprises
- at least one printed circuit board assembly (93), particularly a single printed circuit board assembly (93).

3. Housing according to claim 2, **characterized in that** the window carrier (17) is held by compression between the housing body (3) and the housing cover (18) forming a linkage between the housing body (3) and the housing cover (18).

4. Housing according to any of the preceding claims, **characterized in that** eyelets (2) for guiding and stabilizing the yarn (34) in a measurement gap of a clearer (10) are placed in the window carrier (17) or at the housing body (3) for one part and at the housing cover (18) as the other part.

5. Housing according to any of the preceding claims, **characterized in that** the window carrier (17) carries at least one window on an emission side and at least one window on a receiving side and wherein a yarn guiding device (11) is formed by the window carrier (17) to guide the yarn (34) in a position for illumination between the emission side and the receiving side.

6. Housing according to any of the preceding claims, **characterized in that** at least one of the following applies:
- no sealing compound or seal seals the parts contributing to the housing;
- no glue glues the parts contributing to the housing;
- no electronics are connected to the housing; or
- all electronics are on at least one printed circuit board assembly (93), particularly a single printed circuit board assembly (93).

7. Housing according to any of the preceding claims, **characterized in that** a meandering seal is formed such that at least a first wall (90) and a second wall (92) are positioned and configured such that they run at least together at a seam (77) to be sealed, particularly parallel to each other, wherein both walls (90, 92) at least provide one angular turn to force dust entering the meandering seal and migrating between the at least first wall (90) and second wall (92) to change direction at least once, particularly several times.

8. Housing according to any of the preceding claims, **characterized in that** a meandering sealing is formed such that from a seam (77) of the housing cover (18) sunken into the housing body (3) a wall (90) protrudes upwards into the housing; or
wherein a double meandering sealing (79) is formed such that from a seam (77) of the housing cover (18) sunken into the housing body (3) a wall (90) protrudes upwards into the housing up to a first step, where the wall (90) recesses in a direction from a sidewall of the housing body (3) and protrudes upwards up to at least a second step; or
wherein a double meandering sealing (79) is formed such that from a seam (77) of the housing cover (18) sunken into the housing body (3) a wall (90) protrudes upwards into the housing up to a first step, where the wall (90) recesses in a direction from a sidewall of the housing body (3) and protrudes upwards up to a second step and wherein a second wall (92) is placed next to the first wall (90) forming a valley (91) between them.

9. Housing according to any of the preceding claims, **characterized in that** a meandering sealing is formed such that from a seam (77) of the housing cover (18) sunken into the housing body (3) a wall (90) protrudes upwards into the housing at a position where the window carrier (17) engages with the housing cover (18); or
wherein a meandering sealing is formed such that from a seam (77) of the housing body (3) at a position where the window carrier (17) engages with the housing body (3) a wall (90) protrudes downwards into the housing; or
wherein a double meandering sealing (79) is formed such that from a seam (77) of the housing cover (18) sunken into the housing body (3) a wall (90) protrudes upwards into the housing at a position where the window carrier (17) engages with the housing cover, up to a first step, where the wall recesses in a direction from a sidewall of the housing body (3) and protrudes upwards up to at least a second step; or
wherein a double meandering sealing (79) is formed such that from a seam (77) of the housing body (3) at a position where the window carrier (17) engages with the housing body (3) a wall (90) protrudes downwards into the housing, down to a first step, where the wall recesses in a direction from a sidewall of the housing body (3) and protrudes downwards down to at least a second step; or
wherein a double meandering sealing (79) is formed such that from a seam of the housing cover (18) sunken into the housing body a first wall protrudes upwards into the housing at a position where the window carrier (17) engages with the housing cover (18), up to a first step, where the wall (90) recesses in a direction from a sidewall of the housing body (3) and protrudes upwards up to a second step and wherein a second wall (92) is placed next to the first wall (90) forming a valley (91) between them; or
wherein a double meandering sealing (79) is formed such that from a seam (77) of the housing body (3) at a position where the window carrier (18) engages with the housing body (3) a first wall (90) protrudes downwards into the housing, down to a first step, where the wall (90) recesses in a direction from a sidewall of the housing body (3) and protrudes downwards down to at least a second step and wherein a second wall (92) is placed next to the first wall (90) forming a valley (91) between them.

10. Housing according to any of the preceding claims, **characterized in that** the housing comprises a button (8) with a light guide (71) for a signaling light source (32), particularly a signaling LED.

11. Method of assembling a housing, particularly according to any of the claims 1 to 10, **characterized by** the following steps of
- providing a housing comprising a housing body (3) as first housing part, a housing cover (18) as second housing part for covering and/or sealing the housing body (3) and a window carrier (17), wherein the window carrier (17) is detachable from the housing for transition to a second configuration in which at least one window carried by the window carrier (17) may be removed with the window carrier (17) from the housing;
- placing optical components in the housing body (3);
- placing a printed circuit board assembly (93) in the housing body (3);
- connecting the optical components and the printed circuit board assembly (93);
- sinking the housing cover (18) into the housing body (3) to form a gravity seal, oriented such that the housing cover (18) forms the bottom of the housing.

12. Method according to claim 11, **characterized in that** no optical (fine) alignment is performed on the optical components to be linked with the printed circuit board assembly (93) except for mounting the optical components to the same plane on the printed circuit board assembly (93) (giving a course alignment).

13. Method according to either of the claims 11 or 12, **characterized in that** a window carrier (17) is placed in the housing body (3).

14. Method according to any of the claims 11 to 13, **characterized in that** alignment structures (16) align the printed circuit board assembly (93) with the optical components.

## Patentansprüche

1. Gehäuse eines Fadenreinigers (10) für eine Textilmaschine, das zum Führen eines Garns (34) konfiguriert ist, um Eigenschaften des Garns (34) optisch zu erfassen, wobei das Gehäuse umfasst:
- mindestens ein erstes Gehäuseteil, das durch einen Gehäusekörper (3) und einen Fensterträger (17) gebildet ist, wobei der Fensterträger (17) in einer ersten Konfiguration an dem ersten Gehäuseteil so konfiguriert und angeordnet ist, dass Licht von einer Lichtquelle (32) durch mindestens eines von einem ersten Fenster (89) zur Beleuchtung eines geführten Garns (34) oder einem zweiten Fenster (19) hindurchtritt, um das Licht von dem geführten Garn (34) zu einem Empfänger zu leiten;
wobei der Fensterträger (17) vom Gehäuse abnehmbar, insbesondere reversibel vom Gehäuse abnehmbar ist, für einen Übergang in eine zweite Konfiguration, insbesondere zum Reinigen mindestens eines Fensters,
einen Gehäusedeckel (18) als zweites Gehäuseteil zum Abdecken und/oder Abdichten des Gehäusekörpers (3), wobei das Gehäuse **dadurch gekennzeichnet ist, dass** es ferner umfasst: eine Schwerkraftdichtung, die durch den in den Gehäusekörper (3) versenkten Gehäusedeckel (18) gebildet ist, der so auszurichten ist, dass der Gehäusedeckel (18) den Boden des Gehäuses bildet.

2. Gehäuse nach Anspruch 1, **dadurch gekennzeichnet, dass** das Gehäuse umfasst
- mindestens eine Leiterplattenbaugruppe (93), insbesondere eine einzelne Leiterplattenbaugruppe (93).

3. Gehäuse nach Anspruch 2, **dadurch gekennzeichnet, dass** der Fensterträger (17) durch Kompression zwischen dem Gehäusekörper (3) und dem Gehäusedeckel (18) gehalten wird, wodurch eine Verbindung zwischen dem Gehäusekörper (3) und dem Gehäusedeckel (18) gebildet wird.

4. Gehäuse nach einem der vorhergehenden Ansprüche, **dadurch gekennzeichnet, dass** Ösen (2) zum Führen und Stabilisieren des Garns (34) in einem Messspalt eines Fadenreinigers (10) zum einen Teil in dem Fensterträger (17) oder an dem Gehäusekörper (3) und zum anderen Teil an dem Gehäusedeckel (18) platziert sind.

5. Gehäuse nach einem der vorhergehenden Ansprüche, **dadurch gekennzeichnet, dass** der Fensterträger (17) mindestens ein Fenster auf einer Emissionsseite und mindestens ein Fenster auf einer Empfangsseite trägt und wobei eine Garnführungsvorrichtung (11) durch den Fensterträger (17) gebildet wird, um das Garn (34) in einer Position zur Beleuchtung zwischen der Emissionsseite und der Empfangsseite zu führen.

6. Gehäuse nach einem der vorhergehenden Ansprüche, **dadurch gekennzeichnet, dass** mindestens eines der Folgenden zutrifft:
- keine Dichtungsmasse oder Dichtung die zum Gehäuse beitragenden Teile abdichtet;
- kein Klebstoff die zum Gehäuse beitragenden Teile verklebt;
- keine Elektronik mit dem Gehäuse verbunden ist; oder
- die gesamte Elektronik sich auf mindestens einer Leiterplattenbaugruppe (93), insbesondere einer einzelnen Leiterplattenbaugruppe (93), befindet.

7. Gehäuse nach einem der vorhergehenden Ansprüche, **dadurch gekennzeichnet, dass** eine Labyrinthdichtung so ausgebildet ist, dass mindestens eine erste Wand (90) und eine zweite Wand (92) so positioniert und konfiguriert sind, dass sie zumindest zusammen an einer abzudichtenden Naht (77), insbesondere parallel zueinander, verlaufen, wobei beide Wände (90, 92) mindestens eine winklige Biegung vorsehen, um Staub, der in die Labyrinthdichtung eindringt und zwischen der mindestens ersten Wand (90) und der zweiten Wand (92) wandert, zu zwingen, die Richtung mindestens einmal, insbesondere mehrmals, zu ändern.

8. Gehäuse nach einem der vorhergehenden Ansprüche, **dadurch gekennzeichnet, dass** eine Labyrinthdichtung so ausgebildet ist, dass von einer Naht (77) des in den Gehäusekörper (3) versenkten Gehäusedeckels (18) eine Wand (90) nach oben in das Gehäuse hineinragt; oder
wobei eine doppelte Labyrinthdichtung (79) so ausgebildet ist, dass von einer Naht (77) des in den Gehäusekörper (3) versenkten Gehäusedeckels (18) eine Wand (90) nach oben in das Gehäuse bis zu einer ersten Stufe hineinragt, wo die Wand (90) in einer Richtung von einer Seitenwand des Gehäusekörpers (3) zurückspringt und bis zu mindestens einer zweiten Stufe nach oben ragt; oder
wobei eine doppelte Labyrinthdichtung (79) so ausgebildet ist, dass von einer Naht (77) des in den Gehäusekörper (3) versenkten Gehäusedeckels (18) eine Wand (90) nach oben in das Gehäuse bis zu einer ersten Stufe hineinragt, wo die Wand (90) in einer Richtung von einer Seitenwand des Gehäusekörpers (3) zurückspringt und bis zu einer zweiten Stufe nach oben ragt und wobei eine zweite Wand (92) neben der ersten Wand (90) angeordnet ist und ein Tal (91) zwischen ihnen bildet.

9. Gehäuse nach einem der vorhergehenden Ansprüche, **dadurch gekennzeichnet, dass** eine Labyrinthdichtung so ausgebildet ist, dass von einer Naht (77) des in den Gehäusekörper (3) versenkten Gehäusedeckels (18) eine Wand (90) an einer Position, an der der Fensterträger (17) mit dem Gehäusedeckel (18) in Eingriff steht, nach oben in das Gehäuse hineinragt; oder
wobei eine Labyrinthdichtung so ausgebildet ist, dass von einer Naht (77) des Gehäusekörpers (3) an einer Position, an der der Fensterträger (17) mit dem Gehäusekörper (3) in Eingriff steht, eine Wand (90) nach unten in das Gehäuse hineinragt; oder
wobei eine doppelte Labyrinthdichtung (79) so ausgebildet ist, dass von einer Naht (77) des in den Gehäusekörper (3) versenkten Gehäusedeckels (18) eine Wand (90) an einer Position, an der der Fensterträger (17) mit dem Gehäusedeckel in Eingriff steht, bis zu einer ersten Stufe nach oben in das Gehäuse hineinragt, wo die Wand in einer Richtung von einer Seitenwand des Gehäusekörpers (3) zurückspringt und bis zu mindestens einer zweiten Stufe nach oben ragt; oder
wobei eine doppelte Labyrinthdichtung (79) so ausgebildet ist, dass von einer Naht (77) des Gehäusekörpers (3) an einer Position, an der der Fensterträger (17) mit dem Gehäusekörper (3) in Eingriff steht, eine Wand (90) nach unten in das Gehäuse bis zu einer ersten Stufe hineinragt, wo die Wand in einer Richtung von einer Seitenwand des Gehäusekörpers (3) zurückspringt und bis zu mindestens einer zweiten Stufe nach unten ragt; oder
wobei eine doppelte Labyrinthdichtung (79) so ausgebildet ist, dass von einer Naht des in den Gehäusekörper (3) versenkten Gehäusedeckels (18) eine erste Wand an einer Position, an der der Fensterträger (17) mit dem Gehäusedeckel (18) in Eingriff steht, bis zu einer ersten Stufe nach oben in das Gehäuse hineinragt, wo die Wand (90) in einer Richtung von einer Seitenwand des Gehäusekörpers (3) zurückspringt und bis zu einer zweiten Stufe nach oben ragt und wobei eine zweite Wand (92) neben der ersten Wand (90) angeordnet ist und ein Tal (91) zwischen ihnen bildet; oder
wobei eine doppelte Labyrinthdichtung (79) so ausgebildet ist, dass von einer Naht (77) des Gehäusekörpers (3) an einer Position, an der der Fensterträger (17) mit dem Gehäusekörper (3) in Eingriff steht, eine erste Wand (90) nach unten in das Gehäuse bis zu einer ersten Stufe hineinragt, wo die Wand (90) in einer Richtung von einer Seitenwand des Gehäusekörpers (3) zurückspringt und bis zu mindestens einer zweiten Stufe nach unten ragt und wobei eine zweite Wand (92) neben der ersten Wand (90) angeordnet ist und ein Tal (91) zwischen ihnen bildet.

10. Gehäuse nach einem der vorhergehenden Ansprüche, **dadurch gekennzeichnet, dass** das Gehäuse einen Knopf (8) mit einem Lichtleiter (71) für eine Signallichtquelle (32), insbesondere eine Signal-LED, umfasst.

11. Verfahren zum Montieren eines Gehäuses, insbesondere nach einem der Ansprüche 1 bis 10, **gekennzeichnet durch** die folgenden Schritte
- Bereitstellen eines Gehäuses, das einen Gehäusekörper (3) als erstes Gehäuseteil, einen Gehäusedeckel (18) als zweites Gehäuseteil zum Abdecken und/oder Abdichten des Gehäusekörpers (3) und einen Fensterträger (17) umfasst, wobei der Fensterträger (17) vom Gehäuse abnehmbar ist für einen Übergang in eine zweite Konfiguration, in der mindestens ein von dem Fensterträger (17) getragenes Fenster mit dem Fensterträger (17) aus dem Gehäuse entfernt werden kann;
- Platzieren von optischen Komponenten im Gehäusekörper (3);
- Platzieren einer Leiterplattenbaugruppe (93) im Gehäusekörper (3);
- Verbinden der optischen Komponenten und der Leiterplattenbaugruppe (93);
- Versenken des Gehäusedeckels (18) in den Gehäusekörper (3), um eine Schwerkraftdichtung zu bilden, die so ausgerichtet ist, dass der Gehäusedeckel (18) den Boden des Gehäuses bildet.

12. Verfahren nach Anspruch 11, **dadurch gekennzeichnet, dass** keine optische (Fein-) Ausrichtung an den mit der Leiterplattenbaugruppe (93) zu verbindenden optischen Komponenten durchgeführt wird, außer dem Montieren der optischen Komponenten auf derselben Ebene auf der Leiterplattenbaugruppe (93) (was eine Grobausrichtung ergibt).

13. Verfahren nach einem der Ansprüche 11 oder 12, **dadurch gekennzeichnet, dass** ein Fensterträger (17) in den Gehäusekörper (3) platziert wird.

14. Verfahren nach einem der Ansprüche 11 bis 13, **dadurch gekennzeichnet, dass** Ausrichtungsstrukturen (16) die Leiterplattenbaugruppe (93) mit den optischen Komponenten ausrichten.

## Revendications

1. Boîtier d'un purgeur (10) pour une machine textile, configuré pour guider un fil (34) afin de détecter optiquement des caractéristiques dans le fil (34), le boîtier comprenant :
- au moins une première partie de boîtier formée par un corps de boîtier (3) et un porte-fenêtre (17), dans lequel le porte-fenêtre (17) est configuré et agencé dans une première configuration au niveau de la première partie de boîtier pour qu'une lumière provenant d'une source de lumière (32) traverse au moins l'une d'une première fenêtre (89) pour éclairer un fil guidé (34) ou d'une seconde fenêtre (19) pour laisser passer la lumière provenant du fil guidé (34) vers un récepteur;
dans lequel le porte-fenêtre (17) est détachable du boîtier, en particulier détachable de manière réversible du boîtier, pour une transition vers une seconde configuration, en particulier pour le nettoyage d'au moins une fenêtre,
un couvercle de boîtier (18) en tant que seconde partie de boîtier pour recouvrir et/ou sceller le corps de boîtier (3), le boîtier étant **caractérisé en ce qu'**il comprend en outre : un joint par gravité formé par le couvercle de boîtier (18) encastré dans le corps de boîtier (3) pour être orienté de telle sorte que le couvercle de boîtier (18) forme le fond du boîtier.

2. Boîtier selon la revendication 1, **caractérisé en ce que** le boîtier comprend
- au moins un ensemble de carte de circuit imprimé (93), en particulier un unique ensemble de carte de circuit imprimé (93).

3. Boîtier selon la revendication 2, **caractérisé en ce que** le porte-fenêtre (17) est maintenu par compression entre le corps de boîtier (3) et le couvercle de boîtier (18) formant une liaison entre le corps de boîtier (3) et le couvercle de boîtier (18).

4. Boîtier selon l'une quelconque des revendications précédentes, **caractérisé en ce que** des œillets (2) pour guider et stabiliser le fil (34) dans un entrefer de mesure d'un purgeur (10) sont placés dans le porte-fenêtre (17) ou au niveau du corps de boîtier (3) pour une partie et au niveau du couvercle de boîtier (18) pour l'autre partie.

5. Boîtier selon l'une quelconque des revendications précédentes, **caractérisé en ce que** le porte-fenêtre (17) porte au moins une fenêtre côté émission et au moins une fenêtre côté réception et dans lequel un dispositif de guidage de fil (11) est formé par le porte-fenêtre (17) pour guider le fil (34) dans une position d'éclairage entre le côté émission et le côté réception.

6. Boîtier selon l'une quelconque des revendications précédentes, **caractérisé en ce qu'**au moins l'un des points suivants s'applique :
- aucun composé d'étanchéité ou joint ne scelle les pièces contribuant au boîtier ;
- aucune colle ne colle les pièces contribuant au boîtier ;
- aucune électronique n'est connectée au boîtier ; ou
- toute l'électronique se trouve sur au moins un ensemble de carte de circuit imprimé (93), en particulier un unique ensemble de carte de circuit imprimé (93).

7. Boîtier selon l'une quelconque des revendications précédentes, **caractérisé en ce qu'**un joint en labyrinthe est formé de telle sorte qu'au moins une première paroi (90) et une seconde paroi (92) sont positionnées et configurées de manière à s'étendre au moins ensemble au niveau d'un joint (77) à sceller, en particulier parallèlement l'une à l'autre, dans lequel les deux parois (90, 92) fournissent au moins un changement de direction angulaire pour forcer la poussière entrant dans le joint en labyrinthe et migrant entre ladite au moins une première paroi (90) et la seconde paroi (92) à changer de direction au moins une fois, en particulier plusieurs fois.

8. Boîtier selon l'une quelconque des revendications précédentes, **caractérisé en ce qu'**un joint en labyrinthe est formé de telle sorte qu'à partir d'un joint (77) du couvercle de boîtier (18) encastré dans le corps de boîtier (3), une paroi (90) fait saillie vers le haut dans le boîtier ; ou dans lequel un double joint en labyrinthe (79) est formé de telle sorte qu'à partir d'un joint (77) du couvercle de boîtier (18) encastré dans le corps de boîtier (3), une paroi (90) fait saillie vers le haut dans le boîtier jusqu'à un premier gradin, où la paroi (90) est en retrait dans une direction depuis une paroi latérale du corps de boîtier (3) et fait saillie vers le haut jusqu'à au moins un second gradin ; ou
dans lequel un double joint en labyrinthe (79) est formé de telle sorte qu'à partir d'un joint (77) du couvercle de boîtier (18) encastré dans le corps de boîtier (3), une paroi (90) fait saillie vers le haut dans le boîtier jusqu'à un premier gradin, où la paroi (90) est en retrait dans une direction depuis une paroi latérale du corps de boîtier (3) et fait saillie vers le haut jusqu'à un second gradin et dans lequel une seconde paroi (92) est placée à côté de la première paroi (90) formant une vallée (91) entre elles.

9. Boîtier selon l'une quelconque des revendications précédentes, **caractérisé en ce qu'**un joint en labyrinthe est formé de telle sorte qu'à partir d'un joint (77) du couvercle de boîtier (18) encastré dans le corps de boîtier (3), une paroi (90) fait saillie vers le haut dans le boîtier à une position où le porte-fenêtre (17) s'engage avec le couvercle de boîtier (18) ; ou
dans lequel un joint en labyrinthe est formé de telle sorte qu'à partir d'un joint (77) du corps de boîtier (3) à une position où le porte-fenêtre (17) s'engage avec le corps de boîtier (3), une paroi (90) fait saillie vers le bas dans le boîtier ; ou
dans lequel un double joint en labyrinthe (79) est formé de telle sorte qu'à partir d'un joint (77) du couvercle de boîtier (18) encastré dans le corps de boîtier (3), une paroi (90) fait saillie vers le haut dans le boîtier à une position où le porte-fenêtre (17) s'engage avec le couvercle de boîtier, jusqu'à un premier gradin, où la paroi est en retrait dans une direction depuis une paroi latérale du corps de boîtier (3) et fait saillie vers le haut jusqu'à au moins un second gradin ; ou
dans lequel un double joint en labyrinthe (79) est formé de telle sorte qu'à partir d'un joint (77) du corps de boîtier (3) à une position où le porte-fenêtre (17) s'engage avec le corps de boîtier (3), une paroi (90) fait saillie vers le bas dans le boîtier, jusqu'à un premier gradin, où la paroi est en retrait dans une direction depuis une paroi latérale du corps de boîtier (3) et fait saillie vers le bas jusqu'à au moins un second gradin ; ou
dans lequel un double joint en labyrinthe (79) est formé de telle sorte qu'à partir d'un joint du couvercle de boîtier (18) encastré dans le corps de boîtier, une première paroi fait saillie vers le haut dans le boîtier à une position où le porte-fenêtre (17) s'engage avec le couvercle de boîtier (18), jusqu'à un premier gradin, où la paroi (90) est en retrait dans une direction depuis une paroi latérale du corps de boîtier (3)et fait saillie vers le haut jusqu'à un second gradin et dans lequel une seconde paroi (92) est placée à côté de la première paroi (90) formant une vallée (91) entre elles ; ou
dans lequel un double joint en labyrinthe (79) est formé de telle sorte qu'à partir d'un joint (77) du corps de boîtier (3) à une position où le porte-fenêtre (18) s'engage avec le corps de boîtier (3), une première paroi (90) fait saillie vers le bas dans le boîtier, jusqu'à un premier gradin, où la paroi (90) est en retrait dans une direction depuis une paroi latérale du corps de boîtier (3) et fait saillie vers le bas jusqu'à au moins un second gradin et dans lequel une seconde paroi (92) est placée à côté de la première paroi (90) formant une vallée (91) entre elles.

10. Boîtier selon l'une quelconque des revendications précédentes, **caractérisé en ce que** le boîtier comprend un bouton (8) avec un guide de lumière (71) pour une source de lumière de signalisation (32), en particulier une LED de signalisation.

11. Procédé d'assemblage d'un boîtier, en particulier selon l'une quelconque des revendications 1 à 10, **caractérisé par** les étapes suivantes consistant à :
- fournir un boîtier comprenant un corps de boîtier (3) en tant que première partie de boîtier, un couvercle de boîtier (18) en tant que seconde partie de boîtier pour recouvrir et/ou sceller le corps de boîtier (3) et un porte-fenêtre (17), dans lequel le porte-fenêtre (17) est détachable du boîtier pour une transition vers une seconde configuration dans laquelle au moins une fenêtre portée par le porte-fenêtre (17) peut être retirée avec le porte-fenêtre (17) du boîtier ;
- placer des composants optiques dans le corps de boîtier (3) ;
- placer un ensemble de carte de circuit imprimé (93) dans le corps de boîtier (3) ;
- connecter les composants optiques et l'ensemble de carte de circuit imprimé (93) ;
- enfoncer le couvercle de boîtier (18) dans le corps de boîtier (3) pour former un joint par gravité, orienté de telle sorte que le couvercle de boîtier (18) forme le fond du boîtier.

12. Procédé selon la revendication 11, **caractérisé en ce qu'**aucun alignement optique (fin) n'est effectué sur les composants optiques à lier à l'ensemble de carte de circuit imprimé (93), à l'exception du montage des composants optiques sur le même plan sur l'ensemble de carte de circuit imprimé (93) (donnant un alignement grossier).

13. Procédé selon l'une ou l'autre des revendications 11 ou 12, **caractérisé en ce qu'**un porte-fenêtre (17) est placé dans le corps de boîtier (3).

14. Procédé selon l'une quelconque des revendications 11 à 13, **caractérisé en ce que** des structures d'alignement (16) alignent l'ensemble de carte de circuit imprimé (93) avec les composants optiques.
